# EUROPÄISCHE PATENTANMELDUNG

(11) **EP 4 451 284 A1**
(43) Veröffentlichungstag der Anmeldung: **23.10.2024**
(21) Anmeldenummer: 24171213.2
(22) Anmeldetag: 19.04.2024
(51) Int. Cl.: G16H 40/67, G16H 80/00

(54) **SYSTEM ZUR KOMMUNIKATION VON MEDIZINISCH RELEVANTEN DATEN ZWISCHEN EINER MOBILEN BEHANDLUNGSEINHEIT UND EINEM ZENTRUM FÜR TELEMEDIZINISCHE UNTERSTÜTZUNG**

(30) Priorität: 20.04.2023 DE 102023110012
(71) Anmelder: Universität Leipzig, 04109 Leipzig (DE)
(72) Erfinder: Rockstroh, Max, 04109 Leipzig (DE); Pabst, Tobias, 04109 Leipzig (DE); Neumuth, Thomas, 04109 Leipzig (DE)
(74) Vertreter: Hertin und Partner Rechts- und Patentanwälte PartG mbB

(57) **Zusammenfassung**

Die Erfindung betrifft ein System und ein Verfahren zur Kommunikation von medizinisch relevanten Daten zwischen einer mobilen Behandlungseinheit und einem Zentrum für telemedizinische Unterstützung. Das System umfasst Datenerfassungsgeräte für die Erfassung von medizinisch relevanten Daten. Eine Benutzerschnittstelle ist für die Eingabe und/oder Wiedergabe von medizinisch relevanten Daten vorgesehen. Ein Prozessor ist dafür konfiguriert, eine medizinische Situation zu identifizieren, einen medizinischen Verfahrensschritt und die dafür benötigten Services zu identifizieren, diskrete Feature Level für jeden benötigten Service innerhalb des medizinischen Verfahrensschritts abzurufen, eine Priorisierung der FL entsprechend des medizinischen Verfahrensschrittes und aktuellem Behandlungsstatus des Patienten durchzuführen, einen Qualitätsparameter der verfügbaren Netzwerkverbindung zwischen der Kommunikationseinheit und dem Zentrum für telemedizinische Unterstützung zu identifizieren, zu übermittelnde Services mit einem bestimmten FL auszuwählen und die ausgewählten Services optional auf ein bestimmtes FL zu reduzieren. Eine Kommunikationseinheit ist dafür konfiguriert, die ausgewählten Services zum Zentrum für telemedizinische Unterstützung zu übermitteln und/oder Daten von diesem zu empfangen.

## Beschreibung

Die Erfindung betrifft ein System zur Kommunikation von medizinisch relevanten Daten zwischen einer mobilen Behandlungseinheit und einem Zentrum für telemedizinische Unterstützung.

Das System umfasst Datenerfassungsgeräte für die Erfassung von medizinisch relevanten Daten. Eine Benutzerschnittstelle ist für die Eingabe und/oder Wiedergabe von medizinisch relevanten Daten vorgesehen. Ein Prozessor ist dafür konfiguriert, eine medizinische Situation zu identifizieren, einen medizinischen Verfahrensschritt und die dafür benötigten Services zu identifizieren, ein oder mehrere medizinisch sinnvolle diskrete Feature Level (FL) für jeden benötigten Service innerhalb des medizinischen Verfahrensschritts abzurufen, eine Priorisierung der FL entsprechend des medizinischen Verfahrensschrittes durchzuführen, einen Qualitätsparameter der verfügbaren Netzwerkverbindung zwischen der Kommunikationseinheit in der mobilen Behandlungseinheit und dem Zentrum für telemedizinische Unterstützung zu identifizieren, zu übermittelnde Services mit einem bestimmten FL auszuwählen und die ausgewählten Services optional auf ein bestimmtes FL zu reduzieren. Eine Kommunikationseinheit ist dafür konfiguriert, die ausgewählten Services zum Zentrum für telemedizinische Unterstützung zu übermitteln und/oder Daten von diesem zu empfangen.

### Hintergrund und Stand der Technik

Die Erfindung betrifft das technische Gebiet der Telemedizin.

Telemedizin kann für den Austausch medizinischer Informationen von einem Ort zu einem anderen mittels elektronischer Kommunikation genutzt werden. Dies birgt insbesondere für Patienten, die außerhalb von Krankenhäusern behandelt werden, ein großes Potential. Mittels Telemedizin können vorteilhaft Experten aus verschiedenen medizinischen Bereichen trotz geografischer Entfernung bei der Behandlung eines Patienten unterstützen. Medizinisches Fachwissen kann somit schnell zu den Patienten gelangen und die Behandlungsergebnisse verbessern.

Eine schnelle medizinische Beratung ist in der Notfallmedizin unentbehrlich, da sowohl die Geschwindigkeit als auch Korrektheit medizinischer Entscheidungen kritisch für das Überleben oder die Genesung eines Patienten sind. Die Fahrt in einem Rettungswagen stellt einen wichtigen Abschnitt im Behandlungsverlauf dar.

Vor allem in ländlichen Gebieten kann der Weg zu einem Krankenhaus besonders lang sein, so dass während der Fahrt diverse Diagnose- und Behandlungsschritte an dem Patienten durchgeführt werden müssen.

Diese medizinischen Verfahrensschritte werden in der Regel nicht von Fachärzten, sondern vom Personal im Rettungswagen durchgeführt. Das Personal benötigt daher möglicherweise Anleitung oder Supervision bei der Notfallversorgung des Patienten auf dem Weg zum Krankenhaus. Es wäre daher besonders vorteilhaft, dem Personal die erforderliche Anleitung über die Kommunikation mit einem Zentrum für telemedizinische Unterstützung zu bieten. Dies könnte zum Beispiel über ein Mobilfunknetz geschehen. In dem Zentrum können eine oder mehrere Telemediziner anwesend sein.

Eine Herausforderung bei der Bereitstellung zuverlässiger medizinischer Beratung über große Strecken, insbesondere in ländlichen Gebieten, besteht darin, dass der Telemediziner über die medizinische Situation des Patienten ständig ein möglichst aussagekräftiges Bild haben muss. Der Telemediziner benötigt vorzugsweise so viele relevante Informationen wie möglich, um die Situation stets richtig einschätzen und einen möglichst präzisen medizinischen Rat geben zu können. Eine erfolgte medizinische Anweisung muss auch für das Personal im Rettungswagen klar verständlich sein und vorzugsweise ohne Zeitverzug eingehen. Im Idealfall sollte eine Technik zur Bereitstellung der Telemedizin eine möglichst realistische Simulation der Situation erlauben, in welcher der Telemediziner persönlich im Rettungswagen anwesend wäre, so dass der Telemediziner sämtliche Ereignisse in Echt-Zeit wahrnehmen und bewerten kann.

Ein hierfür wünschenswerter vollständiger Informationsfluss ohne zeitliche Verzögerung kann erschwert werden, wenn die Qualität der Mobilfunkverbindung im Verlauf der Fahrt schwankt. Besonders außerhalb von Städten kann der Rettungswagen in einigen Gegenden einer sehr schwachen Netzleistung (umgangssprachliche "Funklöcher") ausgesetzt sein, in denen wenig bis gar kein Datenvolumen mit dem Zentrum für telemedizinische Unterstützung ausgetauscht werden kann.

Sobald sich die Kommunikationsqualität verbessert, kann es dazu kommen, dass veraltete Informationen beim telemedizinischen Zentrum ankommen, die nicht mehr der aktuellen Situation des Patienten entsprechen oder für eine anstehende Entscheidung nicht die aktuelle Relevanz aufweisen. Die Effektivität von Diagnoseverfahren oder einer medizinischen Behandlung wird hierdurch gefährdet. Eine Frustration beim Personal im Rettungswagen kann zusätzlich das Vertrauen in die telemedizinische Unterstützung verringern.

Weiterhin bauen verschiedene Datenströme (wie Audio-, Video-, Sensordaten) eine Verbindung zwischen einem Endpunkt im Rettungswagen und einem Endpunkt im Zentrum für telemedizinische Unterstützung auf und tauschen darüber Daten aus. Im Falle einer schlechten Verbindungsqualität wird die Stärke jeder einzelnen Verbindung verringert, so dass im negativsten Fall keine Daten übertragen werden können oder die übertragenen Daten von so schlechter Qualität sind, dass sie nicht belastbar bewertet werden können. Eine solche Situation ist zu vermeiden, um zu gewährleisten, dass eine potentiell lebensrettende fachärztliche Versorgung stets bereitgestellt werden kann.

Mögliche Lösungsansätze im Stand der Technik basieren derzeit insbesondere auf der Reduzierung der übertragenen Datenströme oder der Erhöhung der verfügbaren Netzwerkkapazität.

US10856206B2 bezieht sich auf eine mobile Behandlungseinheit für Schlaganfallpatienten. Die mobile Behandlungseinheit umfasst eine Vorrichtung zum Scannen des Gehirns eines Patienten und zum Senden von Daten an einen entfernten Neuroradiologen. US10856206B2 räumt ein, dass die Qualität von Mobilfunknetzen außerhalb von Wohngebieten sehr variabel ist. Die Lösung von US10856206B2 besteht darin, die verfügbare Bandbreite ständig zu überwachen und die mobile Behandlungseinheit auf der Grundlage von Karten der Netzwerkverfügbarkeit an einen Standort mit besserer Bandbreite zu verlegen. Dabei wird berücksichtigt, dass diese Methode eine Verzögerung der Ankunft des Patienten in einem Krankenhaus bedeuten kann. Die Autoren der US10856206B2 erläutern, dass es von Vorteil sein kann, die Ankunft des Patienten im Krankenhaus etwas zu verzögern, wenn dies eine frühere Behandlung im Krankenwagen ermöglicht. Diese Lösung basiert auf der Erhöhung der verfügbaren Netzwerkkapazität.

Allerdings scheint die vorgeschlagene Lösung Fälle nicht zu berücksichtigen, in denen ein Krankenwagen besonders weite Strecken zurücklegen muss, um ein Krankenhaus zu erreichen, insbesondere in ländlichen Gebieten. Unter solchen Umständen können kleine Anpassungen der Route eines Krankenwagens die verfügbare Netzwerkkapazität nicht wirksam verbessern. Eine deutliche Verlängerung der Zeit bis zum Erreichen eines Krankenhauses durch die Wahl einer Route mit wesentlich besserer Netzabdeckung ist möglicherweise keine Option. Es besteht daher ein Bedarf an einer besseren Lösung zur Optimierung der Qualität der telemedizinischen Unterstützung für das Personal in einem Krankenwagen, wenn die Netzqualität begrenzt ist.

US10534894B2 schlägt eine Methode zur Erhöhung der verfügbaren Netzwerkkapazität durch Abwechslung bei den verwendeten Verbindungstypen vor. Die US10534894B2 beschreibt dabei eine mobile Behandlungseinheit (ein Fahrzeug) zur Behandlung von traumatischen Hirnereignissen wie Verletzungen, Schlaganfällen, Hirnblutungen und dergleichen. Es wurde festgestellt, dass eine medizinische Beratung nur in 40 % der Fälle erfolgreich empfangen werden konnte (siehe Absatz [0066] der US10534894B2). Um die Erfolgsquote zu verbessern, wird in US10534894B2 vorgeschlagen, zwischen einer Satellitenverbindung und einer Mobilfunkverbindung zu wechseln, um Daten zu übertragen, sobald eine der beiden Verbindungen ausfällt. Dazu muss die mobile Behandlungseinheit mit Satellitenantennen ausgestattet werden. Dies ist zum einen technisch aufwendig und kostenintensiv. Eine weitere Herausforderung bei diesem Lösungsansatz ist, dass die Verbindung zumindest kurzzeitig ständig unterbrochen wird, da die mobile Behandlungseinheit eine gewisse Zeit benötigt, um eine neue Verbindung herzustellen. Für eine Behandlungseinheit, die große Entfernungen mit hoher Geschwindigkeit überquert, kann dies häufige Unterbrechungen bedeuten, die zu Datenverlusten führen oder dazu, dass aus veralteten oder weniger relevanten Informationen falsche Schlüsse gezogen werden. Wie in [0139] der US10534894B2 anerkannt wird, führt das ständige Hin- und Herwechseln zwischen alternativen Verbindungen dazu, dass keine Daten gesendet oder empfangen werden.

Um auch eine weniger gute Verbindungsqualität zwischen dem Rettungswagen und dem Zentrum für telemedizinische Unterstützung effektiv zu nutzen, kann es deshalb notwendig sein, die Menge der übertragenen Daten zu reduzieren. Dies kann durch das Abschalten verschiedener Datenströme geschehen, um das System zu entlasten. Dies ist z.B. aus der online Kommunikation bekannt, wo ein Telekonferenz Service automatisch das Webcam-Streaming einiger Benutzer abschaltet, sobald deren Netzwerkverbindung nicht mehr ausreicht, um sowohl Audio- als auch Videostreams zu übertragen.

Im Kontext einer Videokonferenz offenbart die WO2013/188107A1 die Verwendung von allgemeinen Prioritätsmetriken beim Streaming von Videos. Wenn kurzzeitig eine bessere Netzwerkverbindung zur Verfügung steht, kann die Pufferung der kommenden Teile des Videos erhöht werden. Dies kann das Anhalten eines Videos reduzieren und den Eindruck eines kontinuierlichen high-end Live-Streamings aufrechterhalten.

Ein solcher Ansatz ist für die telemedizinische Unterstützung zur Behandlung von Patienten in einem Rettungswagen nicht geeignet. Da sich die Situation des Patienten im Rettungswagen innerhalb von Millisekunden ändern kann und medizinische Ratschläge sofort befolgt werden müssen, ist eine pauschale Bereitstellung von ggf. veralteten Informationen kontraproduktiv. Die Aktualität und medizinische Relevanz der übermittelten Daten hat Vorrang vor der Vermittlung des Eindrucks einer kontinuierlichen high-end Datenübermittlung. Gleichzeitig sind einfache Strategien wie der Vorrang von Text vor Audio oder Audio vor Video für die telemedizinische Betreuung nicht geeignet. Das liegt daran, dass der wichtigste Datenstrom zu einem bestimmten Zeitpunkt derjenige ist, der für die anstehende medizinische Entscheidung erforderlich ist. Dies variiert je nach Patient und dessen aktueller medizinischer Situation. Eine feste Regel, welche Datenströme bei schlechter Konnektivität unterbrochen werden sollten, ist daher nicht sinnvoll.

WO2018/115214A1 offenbart einen alternativen Ansatz für die Übermittlung verschiedener Datenströme zu und von einem Telemediziner, bei dem der medizinische Anwendungsfall berücksichtigt wird. Es wird bestimmt, welche Datenströme zwischen einem entfernten medizinischen Gerät (z.B. bei einem ambulanten Patienten zu Hause) und einem Telemediziner in einem Krankenhaus übertragen werden sollen. Außerdem werden die Datenströme nach Prioritäten eingestuft. Die Prioritäten richten sich nach der Erfahrung des Benutzers (z.B. einer Pflegekraft) und der Art des Diagnose- und Behandlungsschritts, der durchgeführt wird. Die für die Übertragung der ausgewählten Datenströme erforderliche Übertragungsrate wird berechnet und die benötigte Bandbreite wird von einem Netzwerk für die Datenströme angefordert, von denen jeder eine separate Verbindung zum Telemediziner bildet. Eine Kommunikationseinheit weist den Datenströmen die verfügbaren Netzwerkressourcen entsprechend ihrer Prioritäten zu. Die in dem Szenario verfügbaren Netzwerkressourcen sind jedoch relativ konstant, da sich sowohl der Telemediziner als auch das medizinische Gerät konstant an einem Ort befinden. Die Situation des Patienten ist kaum zeitsensitiv und vergleichbar mit dem Transport eines Schwerverletzten im Krankenwagen. Das Verfahren aus WO2018/115214A1 ist daher von geringer Relevanz für den Einsatz einer mobilen Rettungseinheit mit ständig wechselnder Netzwerkqualität und sich potentiell schnell ändernden medizinischen Situationen.

Es besteht daher ein enormer Bedarf an einem System und einem Verfahren zur reibungslosen Kommunikation zwischen einer mobilen Behandlungseinheit und einem Zentrum für telemedizinische Unterstützung, bei dem die sich permanent verändernde Verbindungsqualität entsprechend der konkreten medizinischen Situation optimal genutzt wird. Vorzugsweise sollte das System und das Verfahren die Qualität der Erfahrung (QoE oder "Quality of Experience") des Personals in der mobilen Behandlungseinheit verbessern, sowie die Zuverlässigkeit der übermittelten Daten und der daraus gezogenen Schlüsse erhöhen.

### Aufgabe der Erfindung

Aufgabe der Erfindung ist es, ein System und ein Verfahren bereitzustellen, welche die Kommunikation zwischen einer mobilen Behandlungseinheit und einem Zentrum für telemedizinische Unterstützung unter Berücksichtigung der verfügbaren Netzwerkressourcen verbessern, um dem Personal in der mobilen Behandlungseinheit eine erhöhte QoE anzubieten. Eine weitere Aufgabe der Erfindung ist es, ein System und ein Verfahren bereitzustellen, welche eine sich verändernde Verbindungsqualität optimal ausnutzen, unter Berücksichtigung des sich veränderbaren Behandlungsstatus eines Patienten innerhalb einer medizinischen Situation. Eine weitere Aufgabe der Erfindung ist es, ein System und ein Verfahren bereitzustellen, das die bestmögliche Versorgung eines Patienten in einer mobilen Behandlungseinheit gewährleistet und gleichzeitig ein Krankenhaus mit Informationen versorgt, welche zur Vorbereitung der Ankunft des Patienten erforderlich sind.

### Zusammenfassung der Erfindung

Gelöst wird die Aufgabe durch die Merkmale der unabhängigen Ansprüche. Vorteilhafte Ausgestaltungen der Erfindung sind in den abhängigen Ansprüchen beschrieben.

In einem ersten Aspekt betrifft die Erfindung ein System zur Kommunikation von medizinisch relevanten Daten zwischen einer mobilen Behandlungseinheit und einem Zentrum für telemedizinische Unterstützung. Das System umfasst ein oder mehrere Datenerfassungsgeräte, eine oder mehrere Benutzerschnittstellen, einen Prozessor und eine Kommunikationseinheit in der mobilen Behandlungseinheit. Die Datenerfassungsgeräte sind für die Erfassung von medizinisch relevanten Daten konfiguriert. Die eine oder mehreren Benutzerschnittstellen sind für die Eingabe und/oder Wiedergabe von medizinisch relevanten Daten konfiguriert. Der Prozessor ist dafür konfiguriert:
- anhand von Eingangsdaten von dem einen oder mehreren Datenerfassungsgeräten, von der einen oder mehreren Benutzerschnittstellen und/oder von der Kommunikationseinheit eine medizinische Situation zu identifizieren,
- auf Basis der medizinischen Situation und der Eingangsdaten einen medizinischen Verfahrensschritt und die dafür benötigten Services zu identifizieren,
- ein oder mehrere medizinisch sinnvolle diskrete Feature Levels (FL) für jeden benötigten Service innerhalb des medizinischen Verfahrensschritts abzurufen,
- eine Priorisierung der FL entsprechend dem medizinischen Verfahrensschritt durchzuführen,
- mindestens einen Qualitätsparameter einer verfügbaren Netzwerkverbindung zwischen der Kommunikationseinheit und dem Zentrum für telemedizinische Unterstützung zu identifizieren,
- zu übermittelnde Services mit einem bestimmten FL unter Berücksichtigung der Priorisierung und des Qualitätsparameters der verfügbaren Netzwerkverbindung auszuwählen,
- die ausgewählten Services optional auf ein bestimmtes FL zu reduzieren.

Die Kommunikationseinheit ist dafür konfiguriert ist, die ausgewählten Services zum Zentrum für telemedizinische Unterstützung zu übermitteln und/oder Daten von diesem zu empfangen.

Mit Hilfe des erfindungsgemäßen Systems können medizinisch relevante Daten zwischen einer mobilen Behandlungseinheit und einem Zentrum für telemedizinische Unterstützung besonders zuverlässig und nutzbringend ausgetauscht werden. Insbesondere wird mittels des Systems die verfügbare Netzverbindung vorteilhaft optimal genutzt, wobei in einer sich ändernden medizinischen Situation die jeweils relevantesten Daten an das Zentrum übermittelt werden. Das System stellt sicher, dass die übermittelten Daten aussagekräftig und von medizinischem Nutzen sind. Auf diese Weise kann eine medizinische Diagnose oder eine medizinische Behandlungsempfehlung zuverlässig von der mobilen Behandlungseinheit empfangen werden, wobei die Diagnose oder medizinische Behandlungsempfehlung dem aktuellen Zustand des Patienten entspricht. Gleichzeitig wird die Qualität der Benutzererfahrung verbessert. Das Personal im Rettungswagen kann sich auf die Diagnose und Behandlung des Patienten konzentrieren ohne Ablenkungen und Störungen durch potentielle Kommunikationsstörungen mit dem telemedizinischen Zentrum. Die Fahrzeit, um einen Notfallpatienten in ein Krankenhaus zu bringen, wird somit trotz schwankender Netzqualität optimal genutzt, was die Behandlungsergebnisse für den Patienten verbessert. Hierdurch können auch Patienten, welche in entlegenen Gegenden in eine medizinische Notsituation geraten, bestmöglich versorgt werden.

Die Datenerfassungsgeräte werden vorzugsweise verwendet, um den Patienten zu überwachen und das System (und damit beispielsweise einen Telemediziner) über dessen Zustand kontinuierlich zu informieren. Die Datenerfassungsgeräte können auch zur Unterstützung der Kommunikation zwischen dem Personal in der mobilen Behandlungseinheit und dem Telemediziner eingesetzt werden. Darüberhinaus kann die Benutzerschnittstelle zur Unterstützung dieser Kommunikation verwendet werden, beispielsweise indem das Personal Daten eingeben kann. Diese Daten können die von den Datenerfassungsgeräten aufgezeichneten oder vom System berechneten oder geschätzten Daten überlagern. Die von den Datenerfassungsgeräten und der Benutzerschnittstelle aufgezeichneten Daten können ein Teil der "Eingangsdaten" sein. Zu den Eingangsdaten können auch Daten gehören, die das System empfängt, z.B. medizinische Ratschläge des Telemediziners. Diese Eingangsdaten können vom Prozessor verwendet werden, um auf intelligente Weise Entscheidungen über die Übertragung oder den Empfang von Daten zum und vom Zentrum für telemedizinische Unterstützung zu treffen. Die Eingangsdaten können vom Prozessor auch verwendet werden, um auf intelligente Weise Empfehlungen für die Route der mobilen Behandlungseinheit oder eine Auswahl des anzufahrenden Krankenhauses bereitzustellen. Vorzugsweise können für derartige Empfehlungen beispielsweise die verfügbare Bandbreite oder deren Schwankungen entlang einer Route berücksichtigt werden.

Die Entscheidungsfindung hinsichtlich der Übertragung oder des Empfangs von Daten zum und vom Zentrum für telemedizinische Unterstützung kann in einer Reihe von Schritten erfolgen, die auch vom Prozessor ausgeführt werden können. Zunächst kann der Prozessor die Eingangsdaten verwenden, um eine medizinische Situation zu identifizieren. Dies kann z.B. über einen Algorithmus, eine Referenztabelle oder über eine Eingabe an einer Benutzerschnittstelle geschehen, auf die der Prozessor Zugriff hat. Die Identifizierung der medizinischen Situation ermöglicht es, weitere Entscheidungen, wie die Priorisierung und Reduzierung von Daten, auf medizinisch sinnvolle Weise vorzunehmen. Die medizinische Situation liefert also den Kontext für diese weiteren Entscheidungsschritte. Dies ist eine Verbesserung gegenüber dem Stand der Technik, bei dem nur eine generische Priorisierung stattfindet (z.B. Text vor Audio oder Audio vor Video), die nicht für jeden Patienten und seinen individuellen Zustand geeignet ist. Darüber hinaus kann die anfängliche Identifizierung der medizinischen Situation die möglichen Optionen (z.B. Behandlungsoptionen) eingrenzen, zwischen denen der Prozessor später wählen kann, wodurch sich Größe, Geschwindigkeit und Effizienz des Systems zusätzlich verbessern.

Als nächstes kann der Prozessor den aktuellen oder nächsten medizinischen Schritt auf der Grundlage der medizinischen Situation identifizieren (bestimmen, vorhersagen oder erkennen). Ein "Identifizieren" des aktuellen oder nächsten medizinischen Schritts umfasst vorzugsweise auch die Festlegung des medizinischen Schritts durch das Personal oder durch einen Telemediziner, insbesondere über eine Benutzerschnittstelle. Vorzugsweise kann der Prozessor manuellen Eingaben eines Benutzers Daten über einen medizinischen Verfahrensschritt zuordnen, auf welchen der Prozessor Zugriff hat. Der medizinische Schritt kann zum Beispiel ein Diagnose- oder Behandlungsschritt sein. Durch die Identifizierung des medizinischen Schritts, der in der mobilen Behandlungseinheit erfolgen soll, können die vom System getroffenen Entscheidungen noch stärker auf die Bedürfnisse eines bestimmten Patienten zu einem bestimmten Zeitpunkt angepasst werden. Beispielsweise kann es sein, dass der Patient im Laufe einer Fahrt in der mobilen Behandlungseinheit verschiedenen Diagnose- oder Behandlungsschritten unterzogen werden muss. In der medizinischen Situation eines Polytraumas muss z.B. häufig in einem ersten Diagnoseschritt das Vorhandensein von inneren Blutungen festgestellt werden. Dies kann z.B. durch die Überwachung des Blutdrucks, der Herz- und Atemfrequenz oder durch eine Ultraschalluntersuchung erfolgen. Zu einem späteren Zeitpunkt muss der Patient möglicherweise einer ersten Behandlungsmaßnahme unterzogen werden, wie einer Änderung seiner Position (z. B. Anheben eines Beins) oder der Verabreichung eines Medikaments. Die Messungen und Beobachtungen, die während der verschiedenen Schritte durchgeführt werden, sind unterschiedlich wichtig, wobei die Ultraschalluntersuchung besonders wichtig für den Diagnoseschritt, aber weniger wichtig für den Behandlungsschritt ist. Es ist daher vorteilhaft, dass der Prozessor erkennt, welcher Schritt zum aktuellen Zeitpunkt oder in der Folge durchgeführt wird, um die Datenübertragungsstrategie dementsprechend dynamisch anzupassen.

Auf der Grundlage der Identifizierung der medizinischen Situation und des medizinischen Verfahrensschritts kann der Prozessor die zu einem bestimmten Zeitpunkt benötigten Services ermitteln. Wie obig beschrieben, kann dies zum Beispiel mit Hilfe eines Algorithmus oder einer Referenztabelle geschehen. Der benötigte Service ist vorzugsweise ein Datenstrom, insbesondere ein Datenstrom, der Teil der Eingangsdaten ist. Der Service kann einen Messdatenstrom von einem Datenerfassungsgerät umfassen, wie z.B. Bilddaten von einem Ultraschallgerät. Ebenso kann der Service zum Beispiel auch Audiodaten von einem Mikrofon in der Benutzerschnittstelle enthalten, über die das Personal in der mobilen Behandlungseinheit Fragen stellen oder den Telemediziner auf den neuesten Stand bringen kann. Durch die Identifizierung aller für den medizinischen Prozessschritt benötigten Services kann die Datenübertragungsstrategie des Systems darauf abgestimmt werden. Gleichzeitig können Services, die zu einem bestimmten Zeitpunkt nicht benötigt werden, aus dem Entscheidungsprozess ausgeschlossen werden und müssen keine Prozessorressourcen oder Verbindungsressourcen beanspruchen.

Der Prozessor kann vorzugsweise anschließend für jeden Service, der für einen aktuellen medizinischen Verfahrensschritt benötigt wird, medizinisch sinnvolle Feature Level (FL) abrufen. FL sind vorzugsweise spezifische, diskrete Unterteilungen eines Services, bei welchen eine medizinische Aussagekraft erhalten bleibt. Vorzugsweise verfügt der Prozessor über Mittel, um die medizinisch sinnvollen FL jedes relevanten Service für den aktuellen medizinischen Verfahrensschritt zu beziehen (oder diese zu schätzen). Diese Informationen können zum Beispiel in einem Speicher enthalten oder einer Cloud abgelegt sein, auf die der Prozessor Zugriff hat. Ein medizinisch sinnvolles FL ist vorzugsweise ein FL, bei dem weiterhin noch medizinisch relevante Informationen erkannt werden können. Zum Beispiel kann ein Ultraschallbild verschiedene, medizinisch sinnvolle Auflösungen haben. Diese können verschiedenen diskreten, medizinisch sinnvollen FL entsprechen. Unterhalb einer bestimmten Auflösung (d.h. bei niedrigeren FL) erscheint das Ultraschallbild derart grob, dass die betreffenden Formen anatomisch nicht bewertbar sind. Solche niedrigen Auflösungen sind medizinisch nicht relevant und können daher von der weiteren Entscheidungsfindung ausgeschlossen werden. Alternativ können die niedrigeren FL einem ausgeschnittenen Segment eines Ultraschallbildes entsprechen, das eine Zielregion darstellt. Wenn noch keine solche Zielregion erkannt wurde, sind die niedrigeren FL medizinisch nicht sinnvoll. Sie können daher unberücksichtigt bleiben. Dies kann besonders nützlich sein, da die Verbindungsressourcen nicht für die Übertragung von Daten verbraucht werden, die für eine telemedizinische Beratung nutzlos sind. Darüber hinaus führt die Einteilung der Services in FL zu einer deutlich schnelleren Entscheidungsfindung, was die Effizienz des Systems weiter verbessert.

Der Prozessor kann die medizinisch sinnvollen FL der benötigten Services für einen bestimmten medizinischen Verfahrensschritt priorisieren. Auf diese Weise kann die verfügbare Verbindung optimal genutzt werden, um die erfassten Daten auf der Grundlage des aktuellen medizinischen Verfahrensschritts und dem aktuellen Behandlungsstatus des Patienten an das Zentrum für telemedizinische Unterstützung zu übermitteln, ohne Ressourcen für irrelevante Datenströme zu belegen oder für Datenströme, die nur auf eine Weise übertragen werden können, die keine medizinisch relevante Aussagekraft besitzen.

Die Übertragung von Daten wird dadurch im Hinblick auf die tatsächliche Relevanz und den potentiellen Nutzen für den Patienten rationalisiert. Der Verlust kostbarer Zeit, während ein Telemediziner auf die Übertragung von Daten wartet, wird vermieden. Stattdessen stehen dem Telemediziner mindestens stets jene Daten bereit, welche entsprechend dem jeweiligen Verfahrensschritt für eine telemedizinische Unterstützung kritisch sind. Dies gewährleistet eine Unterstützung in nahezu Echtzeit, wie diese in Notsituationen erforderlich ist. Die gewonnene Zeit kann zudem genutzt werden, z.B. um die Historie des Patienten, Medikamentenallergien und ähnliches eingehender zu überprüfen und entsprechende medizinische Beratung zu leisten.

Der Prozessor kann vorzugsweise mindestens einen Qualitätsparameter für ein verfügbares Netzwerk zwischen der Kommunikationseinheit der mobilen Behandlungseinheit und dem Zentrum für telemedizinische Unterstützung identifizieren. Im Falle eines Mobilfunknetzes kann der Qualitätsparameter zum Beispiel die Bandbreite sein. Diese Information kann auch in den Entscheidungsprozess einfließen, so dass die verfügbare Bandbreite (oder eine andere Netzwerkressource) optimal genutzt wird, und zwar in einer Weise, die dem medizinischen Verfahrensschritt und dem Behandlungsstatus des Patienten entspricht.

Der Prozessor kann im Anschluss für jeden Service ein bestimmtes FL auswählen, das von der mobilen Behandlungseinheit an das Zentrum für telemedizinische Unterstützung übermittelt werden soll. Dabei kann der Prozessor die Priorisierung der FL und die Qualitätsparameter der verfügbaren Netzwerkverbindung berücksichtigen. Bildlich ausgedrückt kann der Prozessor entscheiden, welche Ausführungsformen (bspw. Bildrate, hier entspricht Ausführungsform dem FL) der benötigten Services in die verfügbare Netzkapazität "passen".

In bevorzugten Ausführungsformen erstellt der Prozessor zu diesem Zweck eine optimierte Liste der Services mit jeweils einem bestimmten FL für jeden Service, der übertragen werden soll, wobei die ausgewählte Kombination von Services und ihren jeweiligen FL die verfügbare Kapazität des Netzwerks vollständig oder nahezu vollständig nutzt. Die Auswahl kann beispielsweise so konfiguriert sein, dass mindestens 80 %, insbesondere mindestens 90 % der Kapazität ausgeschöpft wird. Auf diese Weise wird das verfügbare Netz entsprechend dem aktuellen medizinischen Verfahrensschritt und dem Behandlungsstatus des Patienten optimal genutzt.

Ist die aktuelle Kapazität sehr gut, können bspw. alle Services mit ihrem am höchst priorisierten FL übertragen werden, d.h. die optimierte Liste umfasst dann jeweils ein FL (das am höchsten priorisierte) eines jeden Services, der für den aktuellen Verfahrensschritt relevant ist. Falls der Prozessor anhand der verfügbaren Netzverbindung aber feststellt, dass ein Service nur mit einem niedrigeren (medizinisch sinnvollen) FL an das Zentrum für telemedizinische Unterstützung übertragen werden darf, kann der entsprechende Service auf dieses FL reduziert werden. D.h. die optimierte Liste enthält dann von diesem Service ein niedrigeres FL, zum Beispiel kann die Auflösung eines Ultraschallbildes reduziert werden. Die Anpassung der Datenströme, d.h. die technische Reduktion vom höchst priorisierten FL auf ein niedrigeres FL, kann durch einen Reduzierer geschehen, der vorzugsweise eine geeignete Software umfasst.

Alternativ oder zusätzlich können auch die Einstellungen eines Datenerfassungsgerätes (wie Abtastrate, Auflösung, Bitrate usw.) automatisch an das bestimmte FL eines Services angepasst werden. Dies bedeutet vorzugsweise, statt alle Datenströme auf maximaler Detailebene an den Reduzierer zu senden, der die Reduzierung vornimmt, die Reduzierungsfunktionalitäten der Datenerfassungsgeräte selbst zu verwenden. Somit kann der Reduzierungsprozess vorteilhaft dezentralisiert werden, wodurch er schneller und stabiler durchgeführt werden kann. Zudem können die Einstellungen vorzugsweise entsprechend der jeweiligen Verbindungsqualität angepasst werden, insbesondere auch um einen Datenstrom auf ein höheres FL anzupassen. Dies kann zum Beispiel erforderlich sein, wenn einem ausgewählten Service ein hohes FL zugewiesen wurde. Wenn das zugewiesene FL bspw. höher ist als ein eingestelltes Standard-FL des Services, kann eine Erhöhung erforderlich sein, die vorzugsweise durch Anpassung einer Einstellung des betreffenden Datenerfassungsgeräts erreicht wird.

Nachdem die zu übertragenden Services und ihr jeweiliges FL ausgewählt wurden, und nachdem die Services optional auf ihr jeweiliges FL reduziert wurden, überträgt die Kommunikationseinheit die Services an das Zentrum für telemedizinische Unterstützung.

Das medizinische Personal (oder andere Entscheidungskräfte) im Zentrum für telemedizinische Unterstützung kann somit von der mobilen Behandlungseinheit vorteilhaft die relevantesten medizinischen Daten erhalten, die zu einem bestimmten Zeitpunkt empfangen werden können. Auf der Grundlage dieser aussagekräftigen Daten können relevante und aktuelle medizinische Empfehlungen erarbeitet und zurück übermittelt werden.

Die Kommunikationseinheit der mobilen Behandlungseinheit kann zu diesem Zweck Daten vom Zentrum für telemedizinische Unterstützung empfangen. Dies kann zum Beispiel Diagnose- oder Behandlungsempfehlungen umfassen. Die Daten können ebenso Daten über den Patienten enthalten, wie z.B. Alter, Geschlecht, Schwangerschaftsstatus, Allergien, Vorgeschichte usw. Die empfangenen Daten können vorzugsweise als Grundlage für Maßnahmen dienen, die das Personal in der mobilen Behandlungseinheit ergreift, um das Ergebnis für den Patienten zu verbessern. Das erfindungsgemäße System optimiert somit alle verfügbaren Ressourcen, um dem Patienten während der kritischen Zeit des Transports in ein Krankenhaus die beste Behandlung zukommen zu lassen.

Im Sinne der Erfindung sind "medizinisch relevante Daten" vorzugsweise solche, die eine Diagnose- oder Behandlungsentscheidung beeinflussen können. Die medizinische Relevanz kann zwischen verschiedenen Daten in verschiedenen Kontexten variieren. Für die Behandlung des Patienten sind nicht zu jedem Zeitpunkt alle Informationen gleich relevant. Um einen schnellen Überblick über den Patientenzustand zu bekommen, sind Vitalparameter aus einem Patientenmonitor sinnvoll, aber um z.B. die Wirkung eines Medikaments zu beurteilen, ist die Auswertung des Verlaufs einzelner Parameter über die letzten Minuten deutlich sinnvoller. Die medizinische Relevanz ist vorzugsweise immer kontextabhängig und wird vorzugsweise mit Einschränkungen auf einen Verfahrensschritt oder durch eine Intention angegeben.

Im Sinne der Erfindung ist eine "mobile Behandlungseinheit" vorzugsweise eine bewegbare Einrichtung für die Diagnose, Behandlung und/oder den Transport von Patienten. Vorzugsweise ist die mobile Behandlungseinheit ein Fahrzeug, insbesondere ein Krankenwagen oder Rettungswagen. Auch Militärfahrzeuge, Boote und Hubschrauber können als mobile Behandlungseinheiten bezeichnet werden.

Ein "Zentrum für telemedizinische Unterstützung" im Sinne der Erfindung ist vorzugsweise eine Entität, welche medizinische Informationen auswertet, eine medizinische Diagnose oder medizinische Therapie unterstützt und zu diesem Zweck Daten mit der mobilen Behandlungseinheit über eine Kommunikationseinheit, vorzugsweise kabellos, austauschen kann. Das Zentrum kann zum Beispiel in einem Krankenhaus, einer Klinik, einem Schockraum oder an einem anderen Ort, auch international, untergebracht sein oder durch eine solche Einheit gebildet werden. Vorzugsweise wird das Zentrum von fachmedizinischem Personal betrieben. Es kann jedoch auch bevorzugt sein, dass das Zentrum für telemedizinische Unterstützung vollständig automatisiert betrieben wird, z.B. durch ein System mit künstlicher Intelligenz. In weiteren bevorzugten Ausführungsformen ist das Zentrum teilweise automatisiert und wird teilweise von Personal betrieben.

Das Zentrum kann verschiedene Ausprägungen haben. Beispielsweise umfasst das Zentrum eine Kommunikationseinheit, welche durch einen Telemediziner betrieben wird. Vorzugsweise kann das Zentrum auch ein Team von medizinischen Fachkräften beschäftigen oder von ihnen betrieben werden. Die Kommunikationseinheit kann in einem PC, Telefon oder einer oder mehreren anderen Vorrichtungen vorliegen. Der Telemediziner ist vorzugsweise ein Telenotarzt in einem Telemedizinzentrum oder Krankenhaus. Ebenfalls bevorzugt wird eine medizinische Fachkraft oder ein Schockraumteam der mobilen Behandlungseinheit zugewiesen, wobei der Schockraum vorzugsweise als Zentrum für telemedizinische Unterstützung dient. Vorzugsweise können der Telemediziner, das Team und/oder eine künstliche Intelligenz medizinisch relevante Informationen von der mobilen Behandlungseinheit auswerten oder medizinisch relevante Informationen an die mobile Behandlungseinheit senden.

Die medizinische Unterstützung, die das Zentrum leistet, kann ebenfalls viele verschiedene Formen annehmen. In bevorzugten Ausführungsformen der Erfindung umfasst die telemedizinische Unterstützung eine teleärztliche Begleitung, beispielsweise eine Supervision für Aktivitäten, welche in der mobilen Behandlungseinheit stattfinden. In weiteren bevorzugten Ausführungsformen der Erfindung umfasst die telemedizinische Unterstützung teleärztliche Eingriffe. Dazu gehören das Ein- und Ausschalten medizinischer Vorrichtungen, die Fernsteuerung solcher medizinischen Vorrichtungen und die Durchführung medizinischer Diagnose- und Behandlungstätigkeiten innerhalb der mobilen Behandlungseinheit. Solche Tätigkeiten können zum Beispiel von fernsteuerbaren Vorrichtungen, einschließlich Robotern, ausgeführt werden. Ebenso kann die medizinische Unterstützung medizinische Beratung, die Auswertung von Daten, die für die Situation eines Patienten relevant sind, die Bereitstellung einer Diagnose, die Bereitstellung von Diagnose- oder Behandlungsanweisungen für das Personal in der mobilen Behandlungseinheit und die Beantwortung von Fragen des Patienten oder des Personals in der mobilen Behandlungseinheit umfassen.

Es kann ferner bevorzugt sein, wenn die mobile Behandlungseinheit mit mehr als einem Zentrum für telemedizinische Unterstützung verbunden ist, beispielsweise mit einem oder mehreren entfernten Telenotärzten, einer oder mehreren Medical Computation-Clouds und/oder dem Schockraum im Ziel-Krankenhaus. Im Sinne der Erfindung wird als Medical Computation-Cloud vorzugsweise eine Software verstanden, die medizinische Berechnungen, beispielsweise die Analyse von Ultraschalldaten, nicht in der mobilen Behandlungseinheit ausführt. Im Sinne des effizienten Versands von Daten müssen hierbei doppelte Datensendungen vorzugsweise vermieden werden. Deswegen muss bei derVerbindung mit mehreren Zentren für telemedizinische Unterstützung vorzugsweise immer genau ein primärer Kommunikationspartner festgelegt werden. Im Sinne der Telemedizin ist dies vorzugsweise das Zentrum für telemedizinische Unterstützung mit den höchsten Anforderungen an die Kommunikation. Alle anderen Zentren werden im Sinne dieser einen konkreten Verbindung zu sekundären Kommunikationspartner und beziehen Daten ihrerseits statt von der mobilen Einheit vom primären Kommunikationspartner. Telemedizinische Unterstützung durch alle Zentren gleichzeitig ist denkbar.

Im Sinne der Erfindung sind "Eingangsdaten" vorzugsweise alle Datenströme, die als *Inputs* für den Prozessor dienen. Dazu können auch Daten gehören, die zunächst in einem Speicher gespeichert werden, bevor sie vom Prozessor verarbeitet werden können. Die Eingangsdaten können vorzugsweise von den Datenerfassungsgeräten, der Benutzerschnittstelle und/oder der Kommunikationseinheit stammen. Eingangsdaten von den Datenerfassungsgeräten können zum Beispiel erfasste Daten sein, die Aufschluss über den Zustand eines Patienten und der aktuellen Situation oder des aktuellen Verfahrensschrittes geben. Eingangsdaten von der Benutzerschnittstelle können eine menschliche Auswahl einer medizinischen Situation und/oder eines medizinischen Verfahrensschritts darstellen. Die Eingangsdaten von der Benutzerschnittstelle können auch Abfragen an das Zentrum für telemedizinische Unterstützung liefern. Bei den Eingangsdaten der Kommunikationseinheit kann es sich um Daten handeln, die von dem Zentrum für telemedizinische Unterstützung bereitgestellt werden, wie z.B. Bestandsdaten oder medizinische Empfehlungen. Darüber hinaus können weitere Eingangsdaten auch Aufschluss über den Zustand einer Netzwerkverbindung und/oder einen Standort der mobilen Behandlungseinheit geben. Eingangsdaten können sowohl Live-Daten, Vergangenheitsdaten, und/oder Bestandsdaten eines in der mobilen Behandlungseinheit zu behandelnden Patienten umfassen.

Unter "Live-Daten" sind vorzugsweise Daten zu verstehen, welche von einem oder mehreren Datenerfassungsgeräten wie medizinischen Geräten (welche z.B. direkt bei/an einem Patienten Daten erfassen), Kameras oder Bewegungssensoren in der mobilen Behandlungseinheit erfasst und an das erfindungsgemäße System übertragen werden. Live-Daten können zusätzlich zu beispielswese Messdaten alternativ auch Daten umfassen, welche von einem Benutzer über die Benutzerschnittstelle oder einer anderen Eingabeeinheit an das System übermittelt werden, beispielsweise Medikamentengaben. Erfindungsgemäß werden Live-Daten unmittelbar nach ihrer Erfassung und der Übertragung an das erfindungsgemäße System weiter an ein Zentrum für telemedizinische Unterstützung übermittelt. Live-Daten stellen bevorzugt bei ihrer Prozessierung durch das erfindungsgemäße System und ihrer Übermittlung an ein Zentrum für telemedizinische Unterstützung Echtzeit-Daten eines Patienten dar, welcher in der entsprechenden mobilen Behandlungseinheit behandelt wird. Live-Daten können Daten verschiedener Services umfassen.

Vergangenheitsdaten sind erfindungsgemäß Live-Daten welche von einem oder mehreren Datenerfassungsgeräten in der mobilen Behandlungseinheit erfasst und/oder von einem Benutzer über die Benutzerschnittstelle oder einer anderen Eingabeeinheit an das System übertragen werden, welche jedoch innerhalb einer bestimmten Zeitspanne nicht unmittelbar an ein Zentrum für telemedizinische Unterstützung übermittelt werden konnten (z.B. wegen verringerter Netzwerkverbindungsqualität). Vergangenheitsdaten bilden vorzugsweise einen eigenen Service im erfindungsgemäßen System.

Bestandsdaten sind vorzugsweise Daten, welche bezüglich eines in der mobilen Behandlungseinheit zu behandelnden Patienten in einer medizinischen Datenbank und/oder bei einer medizinischen Einrichtung, z.B. einer Krankenkasse, einem Krankenhaus, dem Hausarzt oder der medizinischen Zieleinrichtung der mobilen Behandlungseinheit, vorliegen und der mobilen Behandlungseinheit zur Verfügung gestellt, d.h. übermittelt werden können. Bestandsdaten können frühere Diagnosen oder andere medizinische oder relevante Informationen über den Patienten umfassen, wie beispielsweise Allergien, Schwangerschaft, frühere Operationen, pharmakologische Unverträglichkeiten, aktuelle Medikationen, frühere Herzinfarkte, Herzleiden, Bluthochdruck, Diabetes, Alter, Geschlecht, etc. Bestandsdaten werden vorzugsweise durch das erfindungsgemäße System mittels einer Kommunikationseinheit empfangen. Bestandsdaten bilden vorzugsweise einen eigenen Service im erfindungsgemäßen System.

Der Begriff "Identifikation" bedeutet im Sinne der Erfindung bevorzugt, dass ein semantisches Konzept (medizinische Situation, medizinischer Verfahrensschritt) durch den Prozessor erkannt wird und beispielsweise einem im System, beispielsweise in einem Speicher, hinterlegten Datensatz zugeordnet werden kann. Alternativ oder zusätzlich kann der Datensatz auf einem Server, im Internet und/oder in einer Cloud für das System abrufbar sein.

Im Sinne der Erfindung ist eine "medizinische Situation" vorzugsweise eine kategorische Beschreibung einer Situation, die einen Patienten betrifft. Bei der medizinischen Situation kann es sich beispielsweise um eine Diagnosekategorie handeln, wobei die Kategorie weit (z. B. Polytrauma) oder enger gefasst sein kann (z. B. Rippenbruch vorne links und Beinwunde). Eine medizinische Situation kann auch eine Behandlungskategorie umfassen oder darstellen, z. B. Herz-Lungen-Wiederbelebung (kardiopulmonale Reanimation). Außerdem kann die medizinische Situation eine kategorische Verhaltensbeschreibung eines Patienten sein (z. B. Erbrechen, Krampfanfall). Jede medizinische Situation besteht vorzugsweise aus einer definierten Anzahl von medizinischen Verfahrensschritten. Verfahrensschritte können sowohl nacheinander ausgeführt werden als auch sich gegenseitig unterbrechend. Wird im Kontext der vorliegenden Erfindung beispielsweise ein Verfahrensschritt gewechselt, ist es die Aufgabe des Systems anhand der von den Datenerfassungsgeräten erfassten und/oder durch die Benutzerschnittstelle eingegebenen und/ oder von der Kommunikationseinheit empfangenen Daten die Veränderung zu erkennen und den aktuell zugehörigen Verfahrensschritt zu identifizieren. Unter allen möglichen Verfahrensschritten wird daher einer, zu einem jeweilig aktuellen Zeitpunkt, als der aktuelle Verfahrensschritt erkannt.

Zu jedem Verfahrensschritt gehört eine bestimmte Anzahl von medizinisch sinnvollen Services und Feature Level (FL). Für die Kommunikation medizinisch relevanter Daten zwischen einer mobilen Behandlungseinheit und einem Zentrum für telemedizinische Unterstützung wird vorzugsweise zunächst eine Liste von medizinisch sinnvollen FL mit den für den aktuellen medizinischen Verfahrensschritt bevorzugt zu übertragenden FL, beziehungsweise einer Spannweite solcher FL, erzeugt. Beispielsweise hat ein bestimmter Service die FL 0-9, wobei die FL 0, 4, 5, 6 und 7 des Services anhand des medizinischen Verfahrensschritts ausgewählt werden. Dies bedeutet, dass FL 7 des Services hier am meisten bevorzugt übermittelt werden würde, aber alternativ bei einer verminderten Netzwerkverbindungs-Qualität auch die Übermittlung einer der FL 4-6 bevorzugt wäre. Im Falle einer sehr geringen Netzwerkverbindungs-Qualität würde ein FL 0 gewählt werden. Die Reduktion des Service auf die FL 1-3 ist dagegen - auch bei ausreichender Netzwerkverbindungsqualität - nicht geeignet, da bei den genannten FL keine medizinisch sinnvollen Daten übertragen werden. Im Gegensatz zu den Lösungen gemäß dem Stand der Technik entscheidet das System daher intelligent im medizinischen Kontext und nicht anhand einfacher Metriken wie Text vor Audio, welche FL für eine Übertragung geeignet sind. Anstatt alle Services mit schwacher und ggf. unzureichender Qualität zu übertragen, werden im Bedarfsfall daher eher weniger relevante Services gar nicht übertragen, um die wichtigen Services mit einer entsprechend guten Qualität übertragen zu können. Die FL müssen nicht numerisch identifiziert werden, sondern können auch "hoch", "mittel" und "niedrig" oder "rot", "gelb" und "grün" oder jede andere geeignete Bezeichnung erhalten.

Im Sinne der Erfindung bezeichnet ein "Service" vorzugsweise einen Datenstrom, welcher Informationen über den aktuellen oder vergangenen Zustand eines Patienten, einer mobilen Behandlungseinheit und/oder einer telemedizinischen Unterstützung umfasst. Die Services können Live-Daten umfassen. Diese Live-Daten können von Datenerfassungsgeräten in der mobilen Behandlungseinheit erfasst werden, wobei vorzugsweise Datenströme von verschiedenen Datenerfassungsgeräten als verschiedene Services betrachtet werden. Die Live-Daten können z.B. Daten von einem Pulsoximeter, einem Blutzuckermessgerät, einem Thermometer, einem EKG-Gerät, einem digitalen Stethoskop, einer Kamera oder einem Mikrofon enthalten, ohne darauf beschränkt zu sein. Die Services können auch Daten von einer Benutzerschnittstelle enthalten, die Text, Ton, Bilder oder Videos enthalten können, ohne darauf beschränkt zu sein. Die Benutzerschnittstelle kann als Eingangs- und Ausgangspunkt für Services dienen, die sich auf die Interaktion zwischen einer medizinischen Fachkraft in der mobilen Behandlungseinheit und einer Datenbank, einer künstlichen Intelligenz oder einem Zentrum für telemedizinische Unterstützung beziehen, ohne darauf beschränkt zu sein. Die Benutzerschnittstelle kann zum Beispiel ein Mikrofon enthalten, über das eine medizinische Fachkraft medizinische Fragen stellt oder Informationen zu einer medizinischen Situation liefert. In diesem Sinne können die über das Mikrofon gesendeten Audioinformationen als ein Service betrachtet werden. Ebenso kann ein Service Daten über den Status der mobilen Behandlungseinheit umfassen, wie z.B. ihren geografischen Standort und die geschätzte Zeit bis zum Eintreffen in einer medizinischen Einrichtung.

Ein Service kann in "Feature Level" (FL) unterteilt werden. FL sind vorzugsweise spezifische, diskrete Unterteilungen eines Services. Bei medizinisch sinnvollen FL bleibt eine medizinische Aussagekraft erhalten. Dies ist vorteilhaft, da Services nicht beliebig reduziert, d.h. in ihren Anforderungen für eine Datenübertragung minimiert, werden können um einen medizinisch relevanten Informationsgehalt beizubehalten. Umfassen die Daten eines Services Videos, können einige der Eigenschaften beispielsweise in gewissen Grenzen beliebig reduziert (komprimiert) werden und einen medizinisch relevanten Informationsgehalt beibehalten. Ein Video hat eine Bitraten-Metrik in kbit/s. Verändert sich die Bandbreite in kbit/s, so kann die Videobitrate im gleichen Maße angepasst werden. Dieser Ansatz kann aber nicht bei allen Services, wie z.B.

EKGs, angewandt werden. Bei EKG-Signalen umfassen spezifische Punkte der EKG-Kurve die medizinisch relevanten Details des Services. EKG-Signale benötigen eine angepasste Reduktion. Zum Beispiel werden EKG-Kurven durch eine Funktion in eine völlig andere Form projiziert. Diese hat eine geringere, aber medizinisch sicher definierte Aussagekraft und verbraucht weniger Bandbreite. FL für EKG könnten beispielsweise sein: inaktiv, projiziert oder vollständig, wobei diese FL jeweils unterschiedliche Anforderungen besitzen.

Im Sinne der Erfindung sind "medizinisch sinnvolle Feature Level" vorzugsweise eine Menge von FL, welche eine Teilmenge der FL einer vollständigen Liste aller Services darstellen und anhand eines medizinischen Verfahrensschrittes als sinnvoll eingestuft bzw. ausgewählt werden. Für einen Service werden in der Regel mehrere FL in diese Menge aufgenommen, nämlich das besonders bevorzugte Ziel-FL (welches zumeist einen oder mehrere optimale Netzwerkqualitätsparameter erfordert bzw. eine in ihrer Qualität uneingeschränkte Netzwerkverbindung, z.B. die höchste Bandbreite und/oder den geringsten Jitter) und alle FL, aus welchen bei verminderter Netzwerkverbindungs-Qualität ein FL für einen Service alternativ für die Übertragung ausgewählt werden kann.

Bei einer "Priorisierung" im Sinne der Erfindung wird vorzugsweise aus der Gesamtmenge der medizinisch sinnvollen Feature Levels eine Liste erstellt, in welcher die FL sortiert und/oder mit Prioritäts-Metainformationen versehen aufgeführt werden. Die priorisierte Liste von FL kann eine Sortierung der Services mit ihren jeweiligen FL oder eine Vergabe von Prioritätswerten oder - klassen an die Services mit ihren jeweiligen FL umfassen. Ebenfalls kann die priorisierte Liste in Form einer multi-dimensionalen Bewertung der Services mit ihren jeweiligen FL vorliegen. Durch die Priorisierung wird gesteuert, welche FL im nächsten Schritt anhand der Netzwerkverbindungs-Qualität für eine Übertragung ausgewählt werden. Beispielsweise wird die Übermittlung der Daten des Services X zu einem gegebenen Zeitpunkt als sehr wichtig bewertet, weshalb alle zuvor als medizinisch sinnvoll bewertet und entsprechend ausgewählten FL des Services X höher priorisiert werden als das höchste FL anderer Services.

In einer ersten bevorzugten Ausführungsform wird für die Priorisierung eine multidimensionale, insbesondere dreidimensionale statische Matrix erzeugt, die für jeden Verfahrensschritt der medizinischen Situationen, jedes medizinisch sinnvolle FL jedes Services sowie für Gruppen der Eingangsdaten einen wohldefinierten Prioritätswert enthält. Diese Matrix kann den kompletten Suchraum abdecken und würde eine vollständige Validierung zulassen. Erstellt würde sie vorzugsweise auf Basis von im System oder einer Datenbank hinterlegten Erfahrungsdaten von Medizinern unterschiedlicher Fachbereiche und/oder Experten des Rettungswesens und/oder der Informatik.

Da das Problem der FL-Priorisierung als ein Rucksacksproblem, definiert werden kann, kann eine gemäß der ersten bevorzugten Ausführungsform erstellte Matrix in einer zweiten bevorzugten Ausführungsform durch maschinelles Lernen für die Priorisierung der FL angewendet werden. In dieser Ausführungsform könnte eine Basis-Matrix für eindeutige oder grundlegende Fälle erstellt werden, aus welcher weitere Fälle durch maschinelles Lernen ergänzt bzw. berechnet werden könnten, sodass nur eine reduzierte Matrix mit einer geringeren Datengröße der mobilen Behandlungseinheit zur Verfügung gestellt werden müsste.

Bei einer "Optimierung" im Sinne der Erfindung wird vorzugsweise anhand der priorisierten Liste von FL sowie der Informationen aus der Kommunikationseinheit über die aktuelle Netzwerkverbindungsqualität eine optimierte Liste von FL erstellt, die eine Teilmenge der priorisierten Liste von FL darstellt. Während der Optimierung wird vorzugsweise unter Berücksichtigung der verfügbaren Netzwerkqualität und aktuell benötigen Netzwerkqualität (welche der summierten Anforderungen der FL entspricht) basierend auf der priorisierten Liste der FL eine optimierte Liste erstellt. Die optimierte Liste erlaubt vorzugsweise eine Übertragung der FL entsprechend ihrer Priorisierung und der Netzwerkverbindungsqualität. Hierbei wird die optimierte Liste vorzugsweise durch Lösung eines mehrdimensionalen oder geschachtelten Rucksackproblems erstellt und dabei jeweils genau ein FL für jeden zu übermittelnden Service aus der priorisierten Liste von FL ausgewählt. Die Netzwerkqualität, insbesondere Mobilfunkqualität, setzt sich vorzugsweise aus mehreren Parametern zusammen. Um die skalaren Größen dieser Parameter gut handhaben zu können, werden sie in der Praxis zu Clustern gruppiert. Liegt eine sehr gute Netzwerkqualität vor, ist beispielsweise die Übertragung aller Services mit ihrem höchsten FL denkbar. Liegt eine sehr schlechte Netzwerkqualität vor, muss ggf. jeder Service auf sein minimales FL reduziert werden oder die Services mit den am niedrigsten priorisierten FL vollständig von der Datenübertragung ausgeschlossen werden. Die optimierte Liste der zu übermittelnden Services umfasst vorzugsweise jeweils nur einen Datenstrom pro Service, z.B. für EKG-Daten.

Unter "Reduzierer" im Sinne der Erfindung sind vorzugsweise Komponenten zu verstehen, die den Datenfluss zwischen einem drahtlosen Netzwerk und der mobilen Einheit überwachen und kontrollieren. Sie erhalten vom erfindungsgemäßen System Anweisungen in Form von FL (genau eines pro Service) und reduzieren die Datenströme der Services im Bedarfsfall, sodass diese danach weniger Netzwerk beanspruchen. Der Reduzierer ist vorzugsweise Teil des Prozessors.

Eine "Kommunikationseinheit" im Sinne der Erfindung ist vorzugsweise eine Vorrichtung, die in der Lage ist, Daten über ein beliebiges Netzwerk zu senden und zu empfangen, wobei die Kommunikationseinheit vorzugsweise eine Schnittstelle zwischen elektronischen Geräten in der mobilen Behandlungseinheit und dem Netzwerk bildet. Die Kommunikationseinheit kann ein Gerät für das Senden und Empfangen von Daten umfassen. Zum Beispiel umfasst die Kommunikationseinheit ein Modem, ein Dongle und/oder ein Netzwerkadapter. Die Kommunikationseinheit umfasst bevorzugt zudem eine Netzwerkkomponente, welche innerhalb der mobilen Behandlungseinheit die Datenverbindung in drahtlose Netzwerke, wie öffentliche Mobilfunknetzwerke, überwacht und dem System regelmäßig aktualisierte Statusinformationen bezüglich der Netzwerk-Qualitätsparameter und Netzwerkqualitätsstufen bereitstellt.

Die Kommunikationseinheit ist außerdem bevorzugt dafür konfiguriert, die übertragenen Datenströme zwischen der mobilen Behandlungseinheit und dem Zentrum für telemedizinische Unterstützung entsprechend der priorisierten, optimierten und ggf. reduzierten FL der zu sendenden Services zu selektieren. Erfindungsgemäß kann die Kommunikationseinheit ein oder mehrere Gateways, Routers, Modems, drahtlose Zugangspunkte und/oder Netzwerkkabel, Leitungstreiber, Hubs, Switches, Multilayer-Switches, Repeater, Protokollkonverter, Proxy-Server, Dateiserver, Datenbankserver, Datenspeicher, Firewalls, Session Border Controller, Netzwerkkarten, Netzwerkadressübersetzer, Multiplexer, und/oder (drahtlose) Netzwerkschnittstellencontroller umfassen.

Im Sinne der Erfindung ist eine "Netzwerkqualität" vorzugsweise eine Messung der Nutzbarkeit einer Verbindung zwischen zwei Einheiten für die Übertragung und/oder den Empfang von medizinisch relevanten Daten. Bei der Bestimmung der allgemeinen Netzwerkqualität können mehrere Parameter berücksichtigt werden. Mobile bzw. drahtlose Netze unterliegen kontinuierlichen Schwankungen in der Qualität ihrer Netzwerkverbindung, zumeist in Abhängigkeit von zahlreichen Faktoren wie Geografie, Wetter, Anzahl der Netzwerk-Teilnehmer und der jeweilig verfügbaren Infrastruktur. Eine Mobilfunkverbindung erlaubt häufig, besonders außerhalb urbaner Gebiete, keinen Netzwerk-/ Internetzugriff mit konstanter Verbindungsqualität. Netzwerkqualitätsparameter mobiler Netzwerke, wie Bandbreite und vor allem Latenz, können über eine gewisse räumliche und/oder zeitliche Distanz signifikanten Schwankungen unterliegen. Ist die Netzwerkqualität ungenügend, können beispielsweise im vorliegenden Fall nicht alle Live-Daten, welche in einer mobilen Behandlungseinheit erfasst werden, in Echtzeit an ein Zentrum für telemedizinische Unterstützung übertragen werden.

Im Sinne der Erfindung ist ein "Netzwerkverbindungs-Qualitätsparameter" vorzugsweise ein quantifizierbares Maß für die Qualität einer verfügbaren Verbindung. Vorzugsweise umfassen die Netzwerkverbindungs-Qualitätsparameter die Bandbreite, Latenz, Jitter, Anzahl Hops und Anzahl parallel nutzbarer Routen der Netzwerkverbindung über die Fahrzeit der mobilen Behandlungseinheit. Diese Netzwerkqualitätsparameter können jeweils in verschiedene Netzwerkqualitätsstufen aufgeteilt bzw. untergliedert werden. Um die Zertifizierbarkeit des Systems zu vereinfachen, können die Netzwerkqualitätsparameter lückenlos in Klassen einsortiert werden. Bei der Bandbreite z.B. alle 100kbit/s also 400kbit/s bis 500 kbit/s, 500 kbit/s bis 600 kbit/s etc. Auch ist eine heterogene Einteilung denkbar.

Im Sinne der Erfindung ist ein "Datenerfassungsgerät" eine Vorrichtung zum Messen und/oder Aufnehmen von Daten. Vorzugsweise ist das Datenerfassungsgerät dazu eingerichtet, ein analoges und/oder nicht-elektrisches Eingangssignal in ein elektrisches und/oder digitales Ausgangssignal umzuwandeln. Hierbei kann das Datenerfassungsgerät eine beliebige physikalische Größe (beispielsweise Temperatur, Druck, Lichtintensität, Wellenlänge) als eine elektrische Spannung, Puls und/oder Stromstärke darstellen. Vorzugsweise leitet das Datenerfassungsgerät Ausgangssignale an einen Prozessor, Speicher und/oder eine Kommunikationseinheit weiter. Beispiele von Datenerfassungsgeräten umfassen EKG-Geräte, Puls-Oxymeter, Ultraschallgeräte, Kamera, Bewegungssensoren, aber auch Maus, Tastatur und Touchscreen.

Im Sinne der Erfindung ist eine "Benutzerschnittstelle" vorzugsweise eine Vorrichtung, welche für eine Interaktion zwischen einem Prozessor und einem menschlichen Benutzer konfiguriert ist. Vorzugsweise umfasst die Benutzerschnittstelle eine oder mehrere Eingabeeinheiten, um Signale von einem Benutzer zu empfangen und diese an einen Prozessor weiterzuleiten, und eine oder mehrere Ausgabeeinheiten, um Signale vom Prozessor an den Benutzer weiterzugeben. Bei der Benutzerschnittstelle kann es sich beispielsweise um einen Touchscreen handeln, der die Eingaben eines Benutzers über Berührungsdetektoren empfängt und dem Benutzer visuelle Ausgaben auf dem Touchscreen und/oder einem (zusätzlichen) Bildschirm anzeigt. Als weitere Beispiele kann die Benutzerschnittstelle eine Maus, eine Tastatur, ein Mikrofon, eine Kamera, ein Touchpad, einen Joystick, einen Bewegungsmelder, ein Licht, einen Alarm, einen Lautsprecher, Kopfhörer und/oder Buchsen zum Anschließen dieser Komponenten umfassen.

Im Sinne der Erfindung ist ein "Prozessor" vorzugsweise ein programmierbares Rechenwerk. Vorzugsweise umfasst oder verfügt der Prozessor über eine Software, das die erfindungsgemäßen Verfahrensschritte ausführt. Der Prozessor kann mehrere Prozessoreinheiten umfassen, welche vorzugsweise für die Ausführung verschiedener Funktionen und/oder Verfahrensschritte der Erfindung konfiguriert sind. Die Prozessoreinheiten können vorzugsweise Hardwareeinheiten des Prozessors definieren, die nicht miteinander verdrahtet sein müssen. Ebenfalls können die Prozessoreinheiten Softwaremodule für die Ausführung verschiedener Funktionen und/oder Verfahrensschritte der Erfindung bezeichnen, wobei diese Softwaremodule auf eine oder mehrere Hardwareeinheiten installiert sind. Die Softwaremodule können beispielsweise auf einer oder mehreren Hardwareeinheiten des Prozessors installiert sein, die sich nicht in der mobilen Behandlungseinheit befinden müssen.

Der Prozessor (oder die Prozessoreinheit) verfügt vorzugsweise über einen Speicher und einen Computercode (Software/Firmware) zur Ausführung einer oder mehrerer Verfahrensschritte. Der Prozessor (oder die Prozessoreinheit) kann auch eine programmierbare Leiterplatte, einen Mikrocontroller oder eine andere Vorrichtung zum Empfangen und Verarbeiten von Datensignalen von den Komponenten der Vorrichtung umfassen, beispielsweise von Datenerfassungsgeräten, der Benutzerschnittstelle, der Kommunikationseinheit oder auch von weiteren Prozessoreinheiten. Der Prozessor umfasst vorzugsweise ferner ein computerverwendbares oder computerlesbares Medium, wie eine Festplatte, einen Direktzugriffsspeicher (RAM), einen Festwertspeicher (ROM), einen Flash-Speicher usw., auf dem eine Computersoftware oder ein Code installiert ist. Der Computercode oder die Software zur Ausführung der Verfahrensschritte können in einer beliebigen Programmiersprache oder einer modellbasierten Entwicklungsumgebung geschrieben werden, z. B. in C/C++, C#, Objective-C, Java, Basic/VisualBasic, MATLAB, Python, Simulink, StateFlow, Lab View oder Assembler, ohne auf diese beschränkt zu sein.

Der Begriff "Prozessor ist konfiguriert, um" einen bestimmten Verfahrensschritt auszuführen, wie z.B. die medizinische Situation zu identifizieren, kann eine kundenspezifische oder standardmäßige Software umfassen, die auf dem Prozessor installiert ist und die erforderlichen Rechenschritte initiiert und/oder durchführt. Bei der Software kann es sich insbesondere um ein Softwaremodul handeln, z.B. ein Workflow-Manager.

Die Prozessoreinheiten müssen sich nicht alle innerhalb der mobilen Behandlungseinheit befinden. Vorzugsweise sind alle Prozessoreinheiten für eine drahtgebundene oder drahtlose Kommunikation miteinander konfiguriert. Vorzugsweise befindet sich mindestens eine Prozessoreinheit innerhalb der mobilen Behandlungseinheit und ist so konfiguriert, dass sie Anfragen sendet und Rückmeldungen von den übrigen Prozessoreinheiten empfängt. In einer bevorzugten Ausführungsform der Erfindung ist die mindestens eine Prozessoreinheit innerhalb der mobilen Behandlungseinheit dafür konfiguriert, eine Anfrage für eine externe Prozessoreinheit zu formulieren. Vorzugweise übermittelt eine Kommunikationseinheit die Anfrage an die externe Prozessoreinheit und empfängt von dieser eine Rückmeldung. Die mindestens eine (oder eine weitere) Prozessoreinheit innerhalb der mobilen Behandlungseinheit kann ausgehend von der Rückmeldung einen weiteren Rechenschritt durchführen. Hierdurch können Rechenschritte an eine externe Prozessoreinheit ausgelagert werden, um die technischen und Energieerfordernisse der Geräte in der mobilen Behandlungseinheit zu verringern.

Bevorzugt ist der Prozessor so konfiguriert, dass rechenintensive Schritte vermieden werden, damit die Entscheidungsfindung schnell, robust und zeitlich deterministisch ausgeführt werden kann. Falls nicht alle Rechenschritte effizient ausgeführt werden können, ist der Prozessor vorzugsweise ferner so konfiguriert, dass in einer weiteren bevorzugten Ausführungsform die Software zumindest teilweise in Form eines Cloud-Services bzw. Internet-Dienstes bereitgestellt wird.

In einer bevorzugten Ausführungsform der Erfindung ist eine Software des Prozessors zumindest teilweise in Form eines Cloud-Services bzw. Internet-Dienstes bereitgestellt, wobei der Prozessor eine Datenverarbeitungsanfrage über das Internet an die Cloud übermittelt. Unter Cloud wird im Sinne der Erfindung vorzugsweise ein Berechnungssystem außerhalb der mobilen Behandlungseinheit verstanden. Dieses kann sich in einem weit entfernten Rechenzentrum oder in Form einer Edge-Cloud in einem nahen Mobilfunkmasten befinden, welches im Sinne der Erfindung bevorzugt ist, um die benötigte Zeit für die Datenübertragung gering zu halten. Die Cloud kann in unterschiedlichen Ausführungen als Sharing-Cloud zur Dateiablage und Austauschort dienen, kann als Computation-Cloud Berechnungen des Algorithmus ausführen oder als Medical Computation-Cloud medizinische Berechnungen durchführen.

Auch in diesem Fall liegt eine Software auf dem Prozessor vor, welche Befehle umfasst, den entsprechenden Verfahrensschritt auszuführen. Beispielsweise umfasst die Software auf dem Prozessor ein Befehl, aus den Input-Daten von der Kommunikationseinheit, den Datenerfassungsgeräten und/oder Benutzerschnittstelle eine medizinische Situation zu erkennen.

Die auf einer Prozessoreinheit innerhalb der mobilen Behandlungseinheit installierte Software führt jedoch nicht alle rechenintensive Schritte selbstständig durch, um einen Datensatz bezüglich der medizinischen Situation zu liefern. Stattdessen wird ein Befehl und/oder eine Anfrage an die Cloud zur Bestimmung der medizinischen Situation aus den Input-Datenströmen übermittelt.

Die Software als Cloud-Dienst, welche bevorzugt Befehle umfasst, die Input-Daten mit einer Matrix zu vergleichen und/oder mittels eines Algorithmus zu verarbeiten, verarbeitet die Input-Daten und sendet das Ergebnis zurück an die Prozessoreinheit in der mobilen Behandlungseinheit bzw. an die auf dieser installierten Software. Die Software auf der Prozessoreinheit in der mobilen Behandlungseinheit kann bevorzugt die Ergebnisse weiterverarbeiten.

Wenn im Folgenden bevorzugte Merkmale der Software beschrieben werden, erkennt der Fachmann, dass diese bevorzugt gleichermaßen für eine Software gelten, welche die Schritte vollständig auf einer Prozessoreinheit in der mobilen Behandlungseinheit durchführt, als auch für eine Software, welche einige (bevorzugt rechenintensive) Schritte, wie die Priorisierung der FL, auf eine externe Datenverarbeitungsvorrichtung eines Cloud-Service auslagert, beispielsweise einer Computation-Cloud, Medical Computation-Cloud und/oder Sharing-Cloud. So kann der Cloud-Service zum Beispiel eine Computation-Cloud umfassen, auf welcher komplexere Berechnungen des Algorithmus außerhalb einer Prozessoreinheit in der mobilen Behandlungseinheit durchgeführt werden. Ebenso kann der Cloud-Service eine Medical Computation-Cloud umfassen, wobei eine Software auf der Medical Computation-Cloud dafür konfiguriert ist, medizinische Berechnungen durchzuführen, beispielsweise eine medizinische Bildanalyse. Weiterhin kann es bevorzugt sein, dass der Prozessor auf eine Sharing-Cloud Zugriff hat, um Daten, insbesondere Berechnungsergebnisse, zu lagern und diese mit Benutzern zu teilen, welche über die nötigen Berechtigungen verfügen. Eine entsprechende Software kann auch auf der Sharing Cloud installiert sein, um die Datenfreigabe zu regeln. Ein Fachmann erkennt, dass die vorgesehene medizinisch sinnvolle Übermittlung von Daten an ein Zentrum für telemedizinische Unterstützung als einheitliches Konzept zu verstehen ist, unabhängig davon, welche Schritte der Datenverarbeitung in der mobilen Behandlungseinheit selbst oder durch eine Cloud durchgeführt werden.

In einer bevorzugten Ausführungsform der Erfindung umfasst der Prozessor eine Mehrzahl von Untereinheiten, einen Workflow-Manager, einen Priorisierer, einen Optimierer und/oder einen Reduzierer. Die Untereinheiten sind vorzugsweise funktionelle Untereinheiten, in dem Sinne, dass diese unterschiedliche Funktionen definieren, zu welchen einer oder mehrere Prozessoreinheiten konfiguriert sind. Dabei kann es bevorzugt sein, dass verschiedene funktionelle Untereinheiten hardwareseitig durch ein und dieselbe Prozessoreinheit oder auf verschiedene Prozessoreinheiten realisiert werden. Die Untereinheiten umfassen vorzugsweise mindestens einen Workflow-Manager, mindestens einen Priorisierer, mindestens einen Optimierer und/oder mindestens einen Reduzierer und können vorzugsweise auch Module einer Software betreffen, wobei eine oder mehrere Prozessoreinheiten dafür konfiguriert sind, die Software und mithin die Untereinheiten in Form von Modulen der Software auszuführen. Bevorzugte Merkmale und Funktionen dieser Untereinheiten werden hierin an anderer Stelle detaillierter dargelegt.

In einer bevorzugten Ausführungsform der Erfindung umfasst das System ein oder mehrere medizinische Geräte. Vorzugsweise ist der Prozessor kabelgebunden oder drahtlos mit den medizinischen Geräten verbunden. Vorzugsweise ist der Prozessor so konfiguriert, dass er ein medizinisches Gerät aktiviert, abschaltet oder dessen Einstellungen anpasst. Insbesondere ist der Prozessor dafür konfiguriert, die Einstellungen eines solchen medizinischen Geräts abzulesen und das medizinische Gerät anzusteuern, um seine Einstellungen zu ändern. Ein medizinisches Gerät kann beispielsweise eine Spritzenpumpe (Perfusor) umfassen und eine Einstellung des medizinischen Geräts kann eine Strömungsgeschwindigkeit der Spritzenpumpe umfassen. Es kann bevorzugt sein, dass der Prozessor das medizinische Gerät auf der Grundlage von Daten betreiben kann, die vom Zentrum für telemedizinische Unterstützung über die Kommunikationseinheit empfangen werden oder an einer Benutzerschnittstelle in der mobilen Behandlungseinheit eingegeben werden. Die Eingabe kann vorzugsweise von den Berechtigungen eines Benutzers abhängig sein. Auf diese Weise kann nicht nur eine medizinische Empfehlung über das System empfangen werden, sondern das System kann auch zur Behandlung des Patienten auf der Grundlage der medizinischen Empfehlung, entweder vom Personal in der Behandlungseinheit oder der telemedizinischen Unterstützung, beitragen. Dies kann z.B. der Fall sein, wenn der Prozessor die Anweisung erhält, das medizinische Gerät zu aktivieren, zu deaktivieren oder dessen Einstellungen auf der Grundlage der erhaltenen medizinischen Beratung zu ändern.

Bei "medizinischen Geräten", oder allgemeiner Medizinprodukten, handelt es sich vorzugsweise um Geräte und Produkte im Sinne der EU-Verordnung 2017/745. Vorzugsweise sind die medizinischen Geräte Vorrichtungen, Systeme, Software, Stoffe oder andere Gegenstände, welche für diagnostische und/oder therapeutische Zwecke konfiguriert sind. Bei den diagnostischen und/oder therapeutischen Zwecken handelt es sich beispielsweise um die Diagnose, Verhütung, Überwachung, Vorhersage, Prognose, Behandlung oder Linderung von Krankheiten, Linderung von oder Kompensierung von Verletzungen oder Behinderungen, Untersuchung, Ersatz oder Veränderung der Anatomie oder eines physiologischen oder pathologischen Vorgangs oder Zustands, oder die Gewinnung von Informationen durch die In-vitro-Untersuchung von aus dem menschlichen Körper - auch aus Organ-, Blut- und Gewebespenden - stammenden Proben. Besonders bevorzugt handelt es sich bei den medizinischen Geräten um solche Vorrichtungen, welche Messdatenströme digital (z.B.: per SDC) bereitstellen oder Steuerdaten (z.B. SDC) entgegennehmen und Aktionen (z.B. am Patienten) vornehmen können.

In einer weiteren bevorzugten Ausführungsform der Erfindung umfasst das System eine Vielzahl von Benutzerschnittstellen. Vorzugsweise kann mindestens eine Benutzerschnittstelle innerhalb der mobilen Behandlungseinheit vorhanden sein. Die Benutzerschnittstelle kann ein Display und Lautsprecher für den Empfang von Video- und Audiosignalen von einem Zentrum für telemedizinische Unterstützung umfassen. Die Benutzerschnittstelle kann vorzugsweise auch eine Webcam und ein Mikrofon umfassen, um Video- und Audiosignale an das Zentrum zu senden. Vorzugsweise umfasst die Benutzerschnittstelle außerdem einen Touchscreen, eine Tastatur und/oder eine Maus. Vorzugsweise ist eine weitere Benutzerschnittstelle in dem Zentrum für telemedizinische Unterstützung vorhanden. So kann ein Telemediziner Benutzereingaben machen, die die Entscheidungen des Systems beeinflussen. Wenn der Telemediziner über die entsprechenden Berechtigungen verfügt, kann er auf diese Weise auch eine oder mehrere medizinische Vorrichtungen in der mobilen Behandlungseinheit aus der Ferne steuern.

In einer weiteren bevorzugten Ausführungsform der Erfindung umfasst der Prozessor bzw. eine von dem Prozessor ausgeführte Software einen Workflow-Manager. Der Workflow-Manager verfügt vorzugsweise über eine Matrix, eine Korrelation oder einen Algorithmus, der Beziehungen zwischen den von den Datenerfassungsgeräten erkannten Daten und einer medizinischen Situation bzw. den einzelnen medizinischen Verfahrensschritten definiert. Die Matrix, die Korrelation oder der Algorithmus kann auch Vergangenheitsdaten, Bestandsdaten oder Benutzereingaben für die Identifizierung der medizinischen Situation berücksichtigen. Zum Beispiel kann für jede mögliche Kombination von beispielsweise Puls, Blutdruck, Temperatur, Atemfrequenz und EKG-Werten eine medizinische Situation in einer Matrix angegeben werden. Die Werte können diskretisiert werden. Es kann vorteilhaft sein, wenn die Matrix nicht auf einer Kombination von Werten basiert, sondern auf einer Kombination von Änderungsraten von Werten. Die Matrix kann auf einem Speicher oder in einer Cloud gespeichert werden. Alternativ kann auch ein entscheidungsbasierter Algorithmus verwendet werden, um die notwendigen Datenerfassungsgeräte abzufragen, um zu einer bestimmten medizinischen Situation zu gelangen.

In einer weiteren bevorzugten Ausführungsform der Erfindung hat der Prozessor Zugriff auf eine Vielzahl von Prozessmodellen. Die Prozessmodelle liefern Informationen über verschiedene Abfolgen von medizinischen Verfahrensschritten, die für eine bestimmte medizinische Situation empfohlen werden. Die Prozessmodelle können auch die Services beschreiben, die für jeden medizinischen Verfahrensschritt relevant sind. Die Prozessmodelle beruhen vorzugsweise auf Daten, die von Experten gesammelt wurden. Der Prozessor identifiziert vorzugsweise einen aktuellen oder nachfolgenden medizinischen Verfahrensschritt auf der Grundlage des Prozessmodells. So kann der Prozessor beispielsweise erkennen, dass bestimmte diagnostische Vorrichtungen aktiviert wurden, während ein Patient vom Personal in der mobilen Behandlungseinheit untersucht wird. Auf diese Weise kann das System ein semantisches Verständnis des medizinischen Geschehens erlangen und die vom System getroffenen Entscheidungen werden anhand des aktuellen Verfahrensschrittes unter Berücksichtigung des momentanen Behandlungsstatus des Patienten getroffen.

Für eine medizinische Situation und jeden ihrer entsprechende(n) medizinischen Verfahrensschritt(e) kann ein Prozessmodell bereitgestellt werden oder auf einem Datenspeicher vorliegen, welches die einzelnen Verfahrensschritte beschreibt. Beispielsweise können während der Akutbehandlung eines Polytraumas mehrere Verfahrensschritte durchgeführt werden. Verfahrensschritte können sowohl nacheinander ausgeführt werden als auch sich gegenseitig unterbrechend. Wird im Kontext der vorliegenden Erfindung beispielsweise ein neuer Verfahrensschritt begonnen, ist es die Aufgabe des Systems anhand der von den Datenerfassungsgeräten erfassten und/oder durch die Benutzerschnittstelle eingegebenen und/ oder von der Kommunikationseinheit empfangenen Daten die Veränderung des Verfahrensschrittes zu erkennen und den aktuell zugehörigen Verfahrensschritt zu identifizieren. Unter allen möglichen Verfahrensschritten wird zu einem jeweilig aktuellen Zeitpunkt daher ein Verfahrensschritt als der aktuelle Verfahrensschritt erkannt.

In einer weiteren bevorzugten Ausführungsform der Erfindung ist die Kommunikationseinheit so konfiguriert, dass sie Eingangsdaten, Bestandsdaten, Vergangenheitsdaten, Benutzereingaben und/oder Netzwerkqualitätsdaten an eine Medical Computation-Cloud, Sharing-Cloud oder Computation-Cloud überträgt. Dies kann beispielsweise bei der Durchführung komplexer Berechnungen außerhalb des Prozessors von Vorteil sein. Vorzugweise werden in der Computation Cloud komplexe Berechnung des Algorithmus ausgeführt, während in der Medical Computation Cloud medizinische Berechnung durchgeführt werden. Solche komplexen Berechnungen können die Ausführung von Algorithmen zur Ermittlung der medizinischen Situation und des tatsächlichen/aktuellen medizinischen Verfahrensschritts als auch die Analyse umfangreicher medizinischer Daten umfassen. Die Kapazität und der Energieverbrauch des Prozessors in der mobilen Behandlungseinheit können dadurch reduziert werden.

Weiterhin ist die Verwendung einer Sharing-Cloud vorteilhaft für die gemeinsame Freigabe von Daten für mehrere Benutzer, die Zugriffsrechte auf diese Daten haben. So können die Daten beispielsweise mit dem Zentrum für telemedizinische Unterstützung und mit einem Krankenhaus, in das ein Patient eingeliefert wird, geteilt werden.

In einer bevorzugten Ausführungsform der Erfindung wird die Priorisierung für jeweils eine Vielzahl von FL für jeden benötigten Service mithilfe einer dreidimensionalen Matrix durchgeführt. Hierbei wird vorzugsweise jedem FL eines jeweiligen Services anhand einer Korrelation von (i) FL, (ii) Verfahrensschritt der medizinischen Situation und (iii) Eingangsdaten ein Prioritätswert zugeordnet. Mittels der Korrelation aller drei vorgenannten Bestandteil können dem Telemediziner die Informationen bereitgestellt werden, die er/sie tatsächlich benötigt, um den aktuellen oder bevorstehenden Verfahrensschritt auf der Grundlage der akuten Situation des Patienten in der mobilen Behandlungseinheit zu begleiten und telemedizinische Supervision zu leisten. So können z.B. stark schwankende oder abnormale Messwerte der Datenerfassungsgeräte vorrangig behandelt werden, selbst wenn sie normalerweise für den Verfahrensschritt von geringerer Relevanz sind. Solche Messwerte könnten für die Diagnose oder die Überprüfung der Wirksamkeit einer Behandlung entscheidend sein. Neben den Eingangsdaten der Datenerfassungsgeräte können auch die Bestandsdaten für die Priorisierung entscheidend sein. Unter Berücksichtigung derer kann die Priorisierung der FL auch an besondere Risikofaktoren des Patienten wie Diabetes, Bluthochdruck, Allergien und dergleichen angepasst werden. Die erhaltene telemedizinische Unterstützung kann somit besonders ganzheitlich und sicher sein, da sie die besonderen Bedürfnisse jedes einzelnen Patienten berücksichtigt.

In einer weiteren bevorzugten Ausführungsform der Erfindung ist die Priorisierung durch Benutzereingaben über die eine oder mehreren Benutzerschnittstellen beeinflussbar. Vorzugsweise ersetzen die Benutzereingaben die durch den Priorisierer ausgewählte Feature Level. Vorzugsweise bedeutet dies, dass der Prozessor so konfiguriert ist, dass er den vom Benutzer getroffenen Priorisierungsentscheidungen Vorrang vor den von der Software durchgeführten Entscheidungen gibt.

Auf diese Weise kann die vom System festgelegte Priorisierung vom Personal in der mobilen Behandlungseinheit oder vom Telemediziner geändert werden, ohne dabei das System grundsätzlich außer Kraft zu setzen. Diese Art der Priorisierung berücksichtigt vielmehr das umfangreiche Fachwissen und die Erfahrung des medizinischen Personals, wodurch eine abweichende Entscheidung gerechtfertigt wird. Wenn sich der medizinische Verfahrensschritt beispielsweise in einem Gebiet mit sehr schwacher Netzverbindung plötzlich ändert, muss das Personal im Zweifel nicht darauf warten, dass das System dies erkennt und mit der Übertragung der entsprechenden Services gemäß der Änderung beginnt. Vielmehr kann das Personal das System zwingen, ausgewählte Services vorrangig an das Zentrum für telemedizinische Unterstützung zu übermitteln. Gleichermaßen kann der Telemediziner eine vom System getroffene Priorisierung ändern, beispielsweise über eine Eingabe an einer Benutzerschnittstelle, die mittels der Kommunikationseinheit in der mobilen Behandlungseinheit empfangen wird.

In einer weiteren bevorzugten Ausführungsform der Erfindung erfolgt die Auswahl der FL der zu übermittelnden Services durch Erstellung einer optimierten Liste von FL basierend auf der priorisierten Liste und der aktuell verfügbaren Netzwerkkapazität. Das bedeutet vorzugsweise, dass aus der priorisierten Liste der Services, die in einem bestimmten medizinischen Prozessschritt benötigt werden, eine optimierte Liste von Ausführungsformen (FL) der Services erstellt wird, welche innerhalb der aktuell verfügbaren Netzwerkkapazität versendet werden können. Dies kann zur Folge haben, dass auf einen oder mehrere Services mit den niedrigsten Prioritäten verzichtet wird.

Die optimierte Liste wird vorzugsweise durch Lösung eines mehrdimensionalen oder geschachtelten Rucksackproblems erstellt. Auf diese Weise können die priorisierten FL intelligent ausgewählt werden, um die verfügbare Netzwerkkapazität optimal zu nutzen, z.B. um die verfügbare Netzwerkkapazität auszuschöpfen und gleichzeitig medizinisch sinnvolle Daten zu übertragen. Im Gegensatz zu einem Bottom-up-Ansatz, bei dem Datenströme mit hoher Priorität einen größeren Netzwerkanteil belegen oder bei dem die Priorisierung statisch erfolgt, können Services proaktiv entsprechend dem medizinischen Kontext, der v.a. bestimmt wird durch den aktuellen Verfahrensschritt und den Behandlungsstatus des Patienten, für die Übertragung ausgewählt werden. Services mit niedriger Priorität, welche nicht auf sinnvoller Weise übertragen werden können, können gezielt ausgelassen werden. Dies steht im Gegensatz zu einer statischen Priorisierungsstrategie, bei der die Services mit niedriger Priorität möglicherweise nicht in die verbleibende Netzwerkkapazität passen und daher nicht in einer medizinisch sinnvollen Weise übertragen werden. Eine mehrdimensionale Analyse ermöglicht es hingegen, einen solchen Service durch einen anderen zu ersetzen, der in die verbleibende Netzwerkkapazität mit einem medizinisch sinnvollen FL passt. Vorzugsweise wird für jeden zu übermittelnden Service jeweils ein FL für die Übermittlung ausgewählt.

In einer weiteren bevorzugten Ausführungsform der Erfindung ist der Prozessor für eine Reduktion auf ein ausgewähltes FL für jeden Service konfiguriert. Die "Reduktion" umfasst vorzugsweise eine Reduktion einer Bandbreite, einer Bildrate, einer Auflösung, einer Abtastrate, einer Frequenz, ein Beschneiden und/oder ein Komprimieren der Daten. Die Reduktion kann einen Skalierungsschritt umfassen, ist aber nicht darauf beschränkt (z.B. in dem Fall des Zuschnitts eines Bilds auf eine Zielregion). Die Reduzierung verringert vorzugsweise die von einem Service benötigten Netzwerkressourcen. Wenn alle für einen bestimmten Verfahrensschritt erforderlichen Services mit der verfügbaren Netzwerkkapazität übertragen werden können, ist eine Reduzierung nicht angezeigt, auch wenn die optimierte Liste einigen Services die höchste FL zuweist. Sofern eine Reduzierung ausgewählter Services auf ihre ausgewählten FL nötig ist, können auch bei eingeschränkter Netzwerkqualität mehrere Services an das Zentrum für telemedizinische Unterstützung übertragen werden. Das Zentrum für telemedizinische Unterstützung kann so immer noch die wichtigsten Informationen über den Zustand eines Patienten erhalten. So können relevante und aktuelle medizinische Ratschläge in der mobilen Behandlungseinheit empfangen werden. Sofern sich das Zentrum für die telemedizinische Unterstützung im Zielkrankenhaus befindet oder eine zweite Verbindung von der mobilen Behandlungseinheit zu dem Zielkrankenhaus besteht, kann sich das ambulante Team optimal auf die Aufnahme eines Patienten vorbereiten, indem es z. B. Ausrüstung für eine Bluttransfusion im Voraus bereitstellt. Dies spart Zeit und verbessert die Heilungschancen des Patienten.

In einer weiteren bevorzugten Ausführungsform der Erfindung umfasst der Prozessor bzw. eine von dem Prozessor ausgeführte Software einen Reduzierer für die Reduktion von Services auf ausgewählte FL.

Bei dem Reduzierer kann es sich um eine oder mehreren Prozessoreinheiten handeln, welche für die Reduktion der Services auf ihre ausgewählten FL konfiguriert sind. Alternativ oder zusätzlich kann es sich um ein Softwaremodul oder mehrere Softwaremodule handeln, welche durch den Prozessor oder durch eine Prozessoreinheit ausgeführt werden können. In bevorzugten Ausführungsformen ist der Reduzierer in der Kommunikationseinheit installiert oder bildet eine separate Komponente des erfindungsgemäßen Systems. Hierbei kann es sich um eine Reduktionseinheit der Kommunikationseinheit handeln, welche für die Reduktion der Services auf ihre ausgewählten FL konfiguriert ist. Ebenfalls kann es sich um ein Softwaremodul handeln, welches durch die Kommunikationseinheit oder die weitere Komponente des erfindungsgemäßen Systems ausgeführt werden kann. In einer bevorzugten Ausführungsform umfasst der Reduzierer eine Mehrzahl von Reduktionseinheiten in den Datenerfassungsgeräten. So kann die Reduktion dezentralisiert erfolgen. Vorzugsweise sind alle Reduktionseinheiten mit dem Prozessor so verbunden, dass sie Anweisungen von dem Prozessor erhalten.

In einer weiteren bevorzugten Ausführungsform der Erfindung sind die zu übermittelnden Services aus der folgenden Gruppe ausgewählt: ein Audiogespräch, ein schriftliches Gespräch, ein Foto, eine Fotobeschreibung, ein Video, ein Ultraschallstream, ein CT-Stream, ein holografischer Stream, ein AR-Stream, Pulsmessungen, EKG-Messungen, EIT-Messungen, Blutzuckermessungen, Blutdruckmessungen, Atemalkoholspiegel, Temperaturmessungen, Sauerstoffsättigungsmessungen, CO₂-Ausatemgehaltsmessungen, Herzfrequenzmessungen, Atemfrequenzmessungen und/oder Bestandsdaten, ohne auf diese beschränkt zu sein. Diese Services können dazu dienen, die medizinische Situation des Patienten zu überwachen und/oder die Kommunikation zwischen dem Personal in der mobilen Behandlungseinheit und dem Zentrum für telemedizinische Unterstützung zu unterstützen. Zum Beispiel können Audio- und Video-Services genutzt werden, um Fragen zu stellen oder medizinische Beratung zu erhalten. Eine solche medizinische Beratung kann nützlicher sein, wenn sie als Videogespräch ausgeführt wird, so dass Mimik und Gestik verfolgt werden können. Solche Gestik kann dabei helfen, medizinische Eingriffe zu erklären. Audio- und Videoaufnahmen können mit einem Mikrofon und/oder einer Webcam der Benutzerschnittstelle der mobilen Behandlungseinheit gemacht werden.

Die gleichen oder zusätzliche Audio- und Video-Services können zur Überwachung des Patienten genutzt werden. Solche Services können zum Beispiel Informationen über die verbalen Äußerungen des Patienten über seine Beschwerden liefern. Video kann auch dazu verwendet werden, das Verhalten des Patienten wie Erbrechen, Krampfanfälle, Zittern und ähnliches zu erkennen. In ähnlicher Weise können Video- oder Foto-Services wichtige Informationen über den Status einer sichtbaren Verletzung (Blässe, Rötung, Schwellung, Blutung, Bluterguss usw.) liefern. Weitere Services wie EKG und Atemfrequenz können als vitale Services gelten und den Gesundheitszustand eines Patienten anzeigen. Alle diese Services können dazu dienen, dem Zentrum für telemedizinische Unterstützung so viele Informationen wie möglich über den Zustand des Patienten zu liefern, damit in einer sich ständig ändernden Situation die bestmögliche Versorgung gewährleistet werden kann.

In einer weiteren bevorzugten Ausführungsform der Erfindung ist der Prozessor ferner so konfiguriert, dass er Bestandsdaten von der einen oder mehreren Benutzerschnittstellen, der Kommunikationseinheit oder dem einen oder mehreren Datenerfassungsgeräten empfängt. Vorzugsweise umfassen die Bestandsdaten Stammdaten eines Patienten, Vergangenheitsdaten, eine Fallbeschreibung, eine Anamnese, Risikofaktoren, eine voraussichtliche Ankunftszeit, eine Einsatzhistorie, ein cABCDE-Schema und/oder Berichtsdaten eines Unfalls.

Die Benutzerschnittstelle kann z.B. ein Kartenlesegerät umfassen oder an ein solches anschließbar sein, wobei das Kartenlesegerät in diesem Fall ein Datenerfassungsgerät darstellt.

Das Kartenlesegerät kann verwendet werden, um die Gesundheitskarte eines Patienten zu lesen und Informationen daraus zu gewinnen. Ebenso können die Stammdaten eines Patienten mit Hilfe der Gesundheitskarte von einem Server über die Kommunikationseinheit heruntergeladen werden. Außerdem kann der Prozessor Informationen empfangen, die vom Personal der mobilen Behandlungseinheit in die Benutzerschnittstelle eingegeben werden. Bei diesen Daten kann es sich um Bestandsdaten handeln, die anders als Live-Daten-Services behandelt werden. Das Personal kann zum Beispiel Details zu einem Unfall eingeben. Das Personal kann auch eine Route auswählen, so dass der Prozessor eine Ankunftszeit am Zielkrankenhaus für die mobile Behandlungseinheit berechnen kann. Solche Daten können an das Zentrum für telemedizinische Unterstützung übertragen werden, sofern sie eine ausreichende Priorität haben und in die verfügbare Netzwerkkapazität passen. Andernfalls können die Daten als Teil der Patientenakte für eine spätere Verwendung gespeichert werden.

Für das Personal der mobilen Behandlungseinheit ist es besonders vorteilhaft, diese Bestandsdaten über den Patienten zu erhalten, da sie für Diagnose- und Behandlungsentscheidungen von Bedeutung sein können. Diese Daten können auch vom Prozessor verwendet werden, um die medizinische Situation und den aktuellen oder nachfolgenden medizinischen Verfahrensschritt zu identifizieren. Gleichzeitig ist es für das Personal in der mobilen Behandlungseinheit nützlich, solche Daten eingeben zu können, damit sie an das Zentrum für telemedizinische Unterstützung übermittelt werden können. Alternativ können sie gespeichert und zur Beurteilung der Situation des Patienten verwendet werden, wenn dieser in einem Krankenhaus angekommen ist. In allen Fällen erhält der Patient eine besser informierte Versorgung.

In einer weiteren bevorzugten Ausführungsform der Erfindung umfasst das System einen Netzwerkqualitätsüberwacher zur Überwachung der Qualitätsparameter der verfügbaren Netzwerkverbindung in der mobilen Behandlungseinheit. Vorzugsweise empfängt der Prozessor Daten über den aktuellen Status der Netzwerkverbindung vom Netzwerkqualitätsüberwacher. Auf diese Weise kann die Optimierung und ggf. Reduzierung von Services auf ihre ausgewählten FL auf der Grundlage einer genauen und aktuellen Bewertung der Netzwerkqualität erfolgen. Die wichtigsten Services können so unter Ausnutzung der verfügbaren Netzwerkkapazität übermittelt werden.

Der überwachte Netzwerkqualitätsparameter kann gespeichert und zur Vorhersage einer zukünftigen Netzwerkqualität verwendet werden. Dies kann durch Extrapolation aus den bisherigen Qualitätsparameterwerten geschehen. Gleichzeitig können die Daten des Netzwerkqualitätsüberwachers mit den Daten eines GPS kombiniert werden, um eine Karte des Netzwerkqualitätsparameters zu erstellen. So kann der Prozessor auch so konfiguriert werden, dass er die wahrscheinlich verfügbare Netzwerkqualität auf einer bestimmten Strecke auf der Grundlage von bereits erfassten Netzwerkqualitätsdaten vorhersagt. Diese Vorhersage kann im Laufe der Zeit verbessert werden. Die Auswahl der zu übertragenden Services und ihrer jeweiligen FL kann also auch von der erwarteten zukünftigen Netzwerkqualität beeinflusst werden. Wird zum Beispiel ein Absinken der Netzwerkqualität vorhergesagt, kann ein Service, der nicht sinnvoll auf ein niedrigeres FL reduziert werden kann, aber für die aktuelle Behandlungssituation von zentraler Bedeutung ist, vorübergehend eine höhere Priorität erhalten.

In einer weiteren bevorzugten Ausführungsform der Erfindung ist der Qualitätsparameter der verfügbaren Netzwerkverbindung aus Bandbreite, Latenz, Jitter, Anzahl Hops und/oder Anzahl parallel nutzbarer Routen einer Netzwerkverbindung ausgewählt. Durch die Überwachung der Latenz und/oder des Jitters kann das System die Kapazität des Netzwerks für den Austausch von Live-Daten ermitteln. Diese Qualitätsparameter sind besonders wichtig für den Austausch von Live-Video- oder Audiogesprächen, da kleine Verzögerungen dazu führen können, dass sich die Teilnehmer übersprechen und das Gespräch zerstückeln, was den Informationsfluss erschwert. Die Bandbreite hingegen ist besonders wichtig, um die Kapazität des Netzwerks für den Austausch von Services zu bestimmen, bei denen es sich um einmalige Daten handelt, wie z.B. ein einzelnes Bild oder die Anamnese-Datei eines Patienten.

In bevorzugten Ausführungsformen der Erfindung überwacht der Netzwerkqualitätsüberwacher mindestens zwei, vorzugsweise mindestens drei, besonders bevorzugt mindestens vier verschiedene Netzwerkqualitätsparameter. Es kann bevorzugt sein, dass jeder der überwachten Qualitätsparameter bei der Auswahl, welche Services mit welchem FL übertragen werden, separat berücksichtigt wird. Zu diesem Zweck kann der Prozessor ein oder mehrere mehrdimensionale oder geschachtelte Rucksackprobleme lösen. Die endgültige Auswahl der Services und FL kann vorzugsweise ein Kompromiss zwischen den Lösungen der verschiedenen Rucksackprobleme darstellen.

In einer weiteren bevorzugten Ausführungsform der Erfindung verfügt der Prozessor über eine Netzwerkqualitätskarte. Der Prozessor ist vorzugsweise dafür konfiguriert, mindestens einen Qualitätsparameter der verfügbaren Netzwerkverbindung anhand der Netzwerkqualitätskarte vorherzusagen. Zum Beispiel können bekannte Netzwerkqualitätsparameter und deren Geolokation zu einer Karte zusammengestellt werden. In Kombination mit einer Straßenkarte können so Routenoptionen anhand der prognostizierten Netzwerkqualität für den Transport des Patienten in ein Krankenhaus festgelegt oder vorgeschlagen werden. Auf diese Weise kann die Auswahl der Services und ihrer jeweiligen FL, die an ein Zentrum für telemedizinische Unterstützung zu übermitteln sind, nicht nur auf einer aktuellen Netzwerkqualität basieren, sondern auch auf einer zukünftigen Netzwerkqualität.

Gleichzeitig können Daten aus der Netzwerkqualitätskarte mit Daten aus einer Straßenkarte kombiniert werden, so dass der Prozessor Routenoptionen anhand der prognostizierten Netzwerkqualität für den Transport des Patienten in ein Krankenhaus festlegen oder vorschlagen kann.

In einer weiteren bevorzugten Ausführungsform der Erfindung ist der Prozessor weiterhin dafür konfiguriert ist, eine Fahrtroute der mobilen Behandlungseinheit anhand einer gespeicherten Netzwerkqualitätskarte und/oder der medizinischen Situation zu berechnen, wobei die berechnete Route vorzugsweise über die eine oder mehreren Benutzerschnittstellen angezeigt wird.

Eine oder mehrere berechnete Fahrtrouten werden vorzugsweise dem Personal in der mobilen Behandlungseinheit vorgeschlagen. Die optimierten Fahrtrouten können vorgeschlagen werden, indem sie auf einer Benutzerschnittstelle zur Auswahl angezeigt werden oder indem sie über eine Freisprecheinrichtung beschrieben werden, so dass das Personal in der mobilen Behandlungseinheit eine Fahrtroute mündlich auswählen kann. Die vorgeschlagene Fahrtroute kann einen Kompromiss zwischen Länge, erwarteter Ankunftszeit und/oder Netzwerkqualität darstellen. Vorzugsweise wird die medizinische Situation des Patienten berücksichtigt. Bei der Netzwerkqualität kann es sich um eine durchschnittliche Qualität auf der vorgeschlagenen Fahrtroute handeln. Bei der Berechnung der Route können auch andere Faktoren berücksichtigt werden, wie z.B. die Verkehrs- und Wetterbedingungen, deren Daten von einem Server empfangen werden können.

Vorzugsweise ermittelt der Prozessor vor Fahrtbeginn der mobilen Behandlungseinheit Vorschläge für Fahrtrouten. Der Prozessor kann hierfür vorzugsweise auf ein Navigationssystem zugreifen. Mithilfe der Netzwerkqualitätskarte und den Daten eines Navigationssystems können Vorschläge für Fahrtrouten gemacht werden, die für eine telemedizinische Behandlung während der Fahrt günstig sind. Darüber hinaus stehen dem System und seinen Benutzern vorzugsweise Prognosen zum Einsatz der Services zur Verfügung. Als Beispiel könnte die Benutzerschnittstelle auf Grund einer solchen Prognose den Benutzern darauf hinweisen: "Zwischen Kilometer 5 und 8 ihrer Fahrt können nur Vitaldaten und Audio übertragen werden. Auf einer 5 Kilometer längeren Route ist mit keinen Übertragungseinschränkungen zu rechnen." Ein solcher Hinweis könnte mit Hilfe einer Karte auf einem Bildschirm der Benutzerschnittstelle gezeigt werden. Wird sich in diesem Beispiel für die kürzere Strecke entschieden, kann das System kontrolliert, bevor die Verbindungsqualität sinkt, wichtige Datenübertragungen abschließen und alle Teilnehmer über die bevorstehende Verbindungsänderungen informieren. Während der Fahrt mit schlechter Übertragungsqualität können nicht übertragene Daten gepuffert und im Anschluss übertragen werden.

Die Information über eine bevorzugte Route können in Vorzugsformen auch als verbindliche Navigationsdaten für ein Navigationssystem der mobilen Behandlungseinheit bereitgestellt werden. Im Falle zumindest teilweise autonom fahrender Behandlungseinheiten können die Navigationsdaten in Vorzugsformen auch für eine Steuerung der mobilen Behandlungseinheit genutzt werden.

In einer weiteren bevorzugten Ausführungsform der Erfindung erfolgt die Priorisierung unter Berücksichtigung eines Datendruckwertes der Vergangenheitsdaten und/oder der Bestandsdaten des Patienten. Der Datendruckwert ist vorzugsweise den Vergangenheitsdaten und/oder den Bestandsdaten durch einen Datendruckberechner im Prozessor zugewiesen. Auf diese Weise kann der Prozessor sicherstellen, dass diese Services an das Zentrum übertragen werden, wenn sie wichtig genug sind und eine ausreichende Netzwerkqualität zur Verfügung steht.

Im Sinne der Erfindung beschreibt ein "Datendruck" vorzugsweise das Verhältnis zwischen zu sendenden Bestandsdaten und/oder Vergangenheitsdaten in Bezug auf zukünftige Sendekapazität. Zukünftige Sendekapazität ergibt sich aus der vorhergesagten Verbindungsqualität der noch verbleibenden Fahrtroute der mobilen Behandlungseinheit zum Zielort. Der Datendruck dient dem System als Abstraktion für die Dringlichkeit der Übermittlung der Bestands- und Vergangenheitsdaten zu einem gegebenen Zeitpunkt. Die Berechnung des Datendrucks ermöglicht eine Voraussicht der nächsten bevorzugt zu übermittelnden bzw. zu empfangenden Daten durch das QoE-System. Der errechnete Datendruck wird den Bestandsdaten- und Vergangenheitsdatenservices jeweils als "MetaDatum" ("Datendruck-Wert") hinzugefügt.

Beispielsweise steht ein Datendruckwert = 0 dafür, dass keine der jeweiligen Daten mehr gesendet bzw. empfangen werden müssen. Ein Datendruckwert von > 1 bedeutet vorzugsweise, dass nicht mehr alle Daten gesendet bzw. empfangen werden können. Je höher der Datendruckwert ist, desto mehr Datendruck liegt an. Vorzugsweise werden bei einem Datendruckwert >1 den Bestandsdaten und Vergangenheitsdaten hohe Prioritäten in einer Priorisierungsmatrix zugeordnet, welche aber geringer als die geringste Priorität der Live-Daten sind. In bevorzugten Ausführungsformen der Erfindung sind die möglichen FL des Bestandsdatenservice "nichts", "wenig", "viel" oder "alles". Die FL würden vorzugsweise ausdrücken, wie viel Bandbreite zur Übertragung verwendet würde. In einem beispielhaften Verfahrensschritt "Aufräumen" würde das FL "alles" eine sehr hohe Priorität bekommen, wohingegen in allen anderen Verfahrensschritten das FL "wenig" höher als die anderen Datendruck FL priorisiert wäre.

In einer weiteren bevorzugten Ausführungsform der Erfindung ist der Prozessor dafür konfiguriert, über die Kommunikationseinheit Services zu einem Krankenhaus, in das ein Patient eingeliefert wird, zu übertragen. Hierbei handelt es sich vorzugsweise um Vergangenheitsdaten. Auf diese Weise kann das Krankenhaus medizinische Informationen erhalten, welche zuvor als Live-Daten nicht gesendet werden konnten. Auf diese Weise steht ein möglichst vollständiges Bild über den Behandlungs- und Gesundheitsverlauf des Patienten zur Verfügung. Ein Ziel-Krankenhaus kann sich so auf die Ankunft des Patienten vorbereiten und die anschließende Behandlung anpassen, um z.B. eine Überdosierung oder schädliche Wechselwirkungen zwischen verschiedenen Medikamenten zu vermeiden.

In einem weiteren Aspekt betrifft die Erfindung ein Verfahren zur Kommunikation von medizinisch relevanten Daten zwischen einer mobilen Behandlungseinheit und einem Zentrum für telemedizinische Unterstützung. Das Verfahren umfasst die folgenden Schritte:
- Identifizieren einer medizinischen Situation anhand von Eingangsdaten von einem oder mehreren Datenerfassungsgeräten, von einer oder mehreren Benutzerschnittstellen und/oder von einer Kommunikationseinheit in der mobilen Behandlungseinheit,
- Identifizieren eines medizinischen Verfahrensschrittes und der dafür benötigten Services auf Basis der medizinischen Situation und der Eingangsdaten,
- Abrufen von diskreten medizinisch sinnvollen Feature Levels (FL), für jeden benötigten Service innerhalb des medizinischen Verfahrensschrittes,
- Priorisierung der FL entsprechend des medizinischen Verfahrensschrittes,
- Identifizieren mindestens eines Qualitätsparameters einer verfügbaren Netzwerkverbindung zwischen der Kommunikationseinheit und dem Zentrum für telemedizinische Unterstützung,
- Auswahl der zu übermittelnden Services mit einem bestimmten FL unter Berücksichtigung der Priorisierung und des Qualitätsparameters der verfügbaren Netzwerkverbindung,
- Optionale Reduktion der ausgewählten Services auf ein bestimmtes FL,
- Übermitteln der ausgewählten Services zum Zentrum für telemedizinische Unterstützung und/oder Empfangen von Daten vom Zentrum für telemedizinische Unterstützung,
wobei das Verfahren vorzugsweise durch ein erfindungsgemäßes System durchgeführt wird.

Die Verfahrensschritte insbesondere für die Auswahl der zu übermittelnden Services mit einem bestimmten FL und die optionale Reduktion der ausgewählten Services auf das bestimmte FL sind vorzugsweise computerimplementiert und werden vorzugsweise durch einen Prozessor, wie in Bezug auf das System beschrieben, durchgeführt.

Begriffe wie im Wesentlichen, ungefähr, etwa, ca., annähernd, nahezu usw. beschreiben vorzugsweise einen Toleranzbereich von weniger als ± 20 %, vorzugsweise weniger als ± 10 %, besonders bevorzugt weniger als ± 5 % und insbesondere weniger als ± 1 % und schließen den genauen Wert ein.

Der durchschnittliche Fachmann erkennt, dass technische Merkmale, Definitionen und Vorteile bevorzugter Ausführungsformen des erfindungsgemäßen Systems auch für das erfindungsgemäße Verfahren gelten, und umgekehrt.

### Detaillierte Beschreibung

Im Folgenden soll die Erfindung anhand von Beispielen und Abbildungen näher erläutert werden, ohne auf diese beschränkt zu sein.

### Kurzbeschreibung der Abbildungen

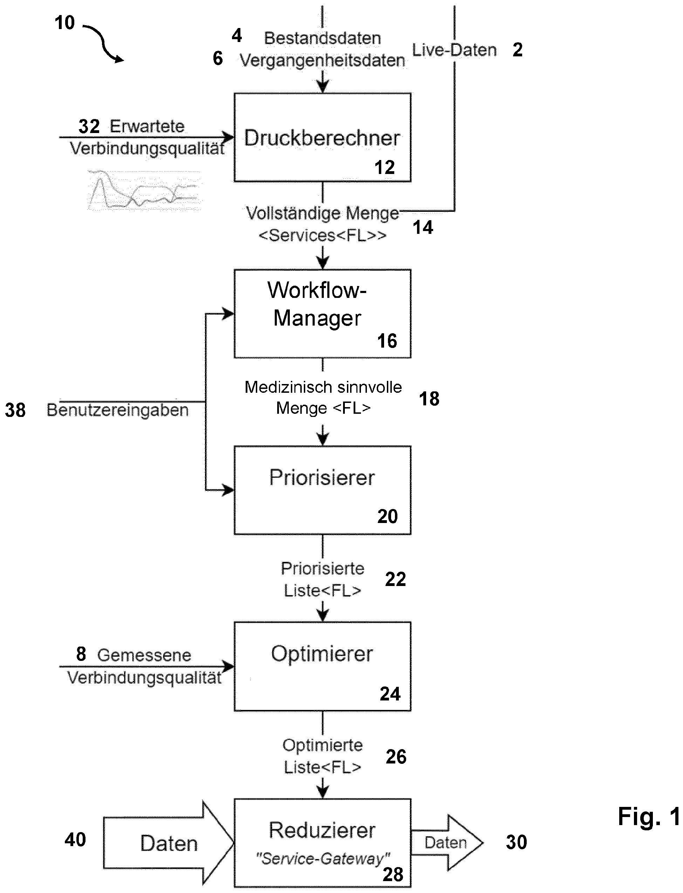

**Fig. 1** ist eine schematische Darstellung eines Verfahrens gemäß einer bevorzugten Ausführungsform der Erfindung.

### Detaillierte Beschreibung der Abbildungen und Ausführungsbeispiele

Ein Beispiel für ein Verfahren, das mit einer bevorzugten Ausführungsform des Systems durchgeführt wurde, wird nachstehend mit Bezugnahme auf Tabelle 1 beschrieben.

**Tabelle 1: Datenübertragung über eine beispielhafte Fahrt**

| **Service** | **FL** | **Schritt 1** | **Schritt 2** | **Schritt 3** | **Schritt 4** | **Schritt 5** | **Schritt 6** | **Schritt 7** | **Schritt 8** |
|---|---|---|---|---|---|---|---|---|---|
| | | Fahrtbeginn | VQ fällt | Blutdru ck sinkt | EKG forciert | Gerät wird eingest ellt | Gerät aktiviert | Patient stabil VQ bessert sich | Alle daten gesend et |
| **4-Kanal-EKG** | 0 - Aus | | | | | | | | x |
| | 1 - 1Hz | | x | | | | | | |
| | 2 - komprimiert | | | x | | x | x | x | |
| | 3 - Live | x | | | x | | | | |
| **Vitaldaten** | 0 - Aus | | | | | | | | x |
| | 1 - 0,3 Hz | | | | x | | | | |
| | 2 - 1 Hz | | x | x | | x | x | x | |
| | 3 - 10 Hz | x | | | | | | | |
| **Audio** | 0 - Aus | | | | | | | | x |
| | 1 - Telefon | | | | x | | | | |
| | 2 - 96kbit/s | | x | x | | x | x | | |
| | 3 - 320kbit/s | x | | | | | | x | |
| **Video** | 0 - Aus | | x | x | x | x | x | | x |
| | 1 - 360p | | | | | | | x | |
| | 2- HD | | | | | | | | |
| | 3-4K | x | | | | | | | |
| **Perfusor** | 0 - Lokal | | x | x | x | | | | x |
| | 1 - Telelnfo | x | | | | x | x | | |
| | 2 - TeleControl | | | | | | | x | |
| **Datendruck der Stammdaten** | | 0,1 | 0,2 | 0,2 | 0,2 | 0,2 | 0,2 | 0,2 | 0 |
| **Stammdaten** | 0 % | | | x | x | x | x | | x |
| | 20 % | | x | | | | | | |
| | 80 % | | | | | | | x | |
| | 100 % | x | | | | | | | |
| **Datendruck des Archive Services** | | 0 | 0,1 | 0,3 | 0,5 | 0,7 | 0,9 | 0,8 | 0,2 |
| **Archive Service** | 0 % | x | x | x | x | x | x | | |
| | 20 % | | | | | | | x | |
| | 80 % | | | | | | | | x |
| | 100 % | | | | | | | | |

Vor Beginn einer Fahrt geht ein Rettungswagenfahrer von einem Patientenraum des Rettungswagens in eine Fahrerkabine. Die Fahrerkabine umfasst eine von mehreren Benutzerschnittstellen des Systems. Der Prozessor schlägt dem Fahrer vor Beginn der Fahrt zwei Fahrtrouten vor. Route α ist 4 Minuten länger als Route β, aber auf Route β kommt es häufig zu Mobilfunkstörungen. Diese Informationen werden auf dem Benutzerschnittstelle in der Fahrerkabine dargestellt. Der Fahrer entscheidet sich nach Absprache (über eine Freisprechanlage) mit einem medizinischen Team für Route β. Das medizinische Team umfasst einen Telenotarzt (in einem Zentrum für telemedizinische Unterstützung), einen Notarzt und einen Notfallsanitäter (im Patientenraum). Der Fahrer wählt diese Option auf dem Touchscreen der Benutzerschnittstelle aus.

Um 12:14 fährt der Rettungswagen ab (Schritt 1 der Tabelle 1). Der Notarzt und Notfallsanitäter kommunizieren mit dem Telenotarzt mittels der Kommunikationseinheit und einer weiteren Benutzerschnittstelle jeweils im Patientenraum und im Zentrum für telemedizinische Unterstützung. Das Team entscheidet sich, auch während der Fahrt ein kontinuierliches Überwachen des Patienten und des Ablaufs der medizinischen Verfahrensschritte an den TNA (Telenotarzt) zur Supervision zu streamen. Der Prozessor identifiziert die notwendigen Services für die gestreamte Kommunikation. Der Prozessor identifiziert auch die medizinische Situation anhand von Daten von Sensoren wie z.B. Überwachungskameras im Patientenraum. In diesem Fall wird ein Polytrauma erkannt. Der Prozessor identifiziert in einem ersten Verfahrensschritt, dass der Patient auf innere Verletzungen untersucht wird. Es wird eine Reihe von Services identifiziert, die für diesen Verfahrensschritt relevant sind. Dies umfasst ein 4-Kanal-EKG-, diverse Vitalparameter sowie Audio- und Videoservices. Während der Fahrt wird - nach Empfehlung des TNA - auch ein EIT-Brustgurt an den Patienten angelegt, um die Lungenfunktion zusätzlich zu überwachen und die erfassten Daten zu übertragen (nicht in der Tabelle ausgeführt). Dieser Service wird manuell durch eine Benutzereingabe des TNA zu der Reihe von Services hinzugefügt, die für den medizinischen Verfahrensschritt relevant sind. Das Personal im Rettungswagen wird hierüber informiert und legt dem Patienten den EIT-Gurt an. Weiterhin wird ein Perfusor (in der Tabelle in Schritt 5 und 6 als "Gerät" bezeichnet) verbunden.

Da die Verbindungsqualität bei Schritt 1 der Fahrt gut ist, werden die Stammdaten des Patienten mit dem höchsten FL übertragen. Da einige Stammdaten auf die Übermittlung warten, entsteht ein Datendruck von 0,1. Zu diesem Zeitpunkt werden noch keine Vergangenheitsdaten akkumuliert, sodass keine archivierten Daten gesendet werden müssen. Der Datendruck ist hierbei 0. Die übrigen Services werden mit der höchsten FL an den Telenotarzt übertragen.

Nach einigen Minuten Fahrt reißt die schnelle Hochfrequenz-Mobilfunk-Verbindung ab (Schritt 2). Der Prozessor prognostiziert eine Verbesserung des Empfangs erst wieder bei Eintritt in den Stadtbereich, ca. 5 Minuten vor Ankunft im Ziel-Krankenhaus. Langwelliges Internet ist weiterhin verfügbar, jedoch steigt dabei die Latenz und die Bandbreite fällt merklich. Das System kappt deswegen das Videosignal und komprimiert Audio-, EKG- und EIT-Services auf jeweils medizinisch sinnvolle FL, sodass alle Services (Datenströme) übertragen werden können. Alle FL werden reduziert.

Um 12:18 (Schritt 3) verschlechtert sich der Zustand des Patienten. Sein Blutdruck sinkt auf 80/50 mmHg. Der Telenotarzt fragt den Notfallsanitäter über Audio nach dem visuellen Zustand des Patienten. Da das Videosignal gekappt wurde, gibt der Notfallsanitäter eine verbale Zustandsbeschreibung des Patienten über die Benutzerschnittstelle (mit Hilfe eines Mikrofons) ein. Die verbale Zustandsbeschreibung reicht dem Telenotarzt nicht aus, um eine fundierte Behandlungsentscheidung zu treffen.

Der Telenotarzt forciert die Priorisierung des EKG-Signals durch eine manuelle Eingabe in seine Benutzerschnittstelle (Schritt 4). Der Prozessor entscheidet nach diesem Eingriff des Benutzers, den EKG-Service wieder mit dem höchsten FL (d.h. unkomprimiert) zu übertragen. Der Prozessor beendet dafür die EIT-Übertragung und reduziert den Audio-Stream weiter auf ein niedrigeres FL. Die Benutzerschnittstelle des Telenotarztes weist ihn darauf hin, die Forcierung zu beenden, wenn sie nicht mehr benötigt wird.

Nach ungefähr 30 Sekunden liegt dem Telenotarzt ein ausreichender Ausschnitt des EKG vor und er kann mit den zuvor erhaltenen Daten weitere Behandlungsentscheidungen treffen. Er hebt die EKG-Forcierung auf und empfiehlt, eine Flussrate des Medikaments an der Spritzenpumpe auf einen neuen Wert einzustellen. Der Telenotarzt informiert den Notarzt und Notfallsanitäter über die Umstellung der Flussrate und den Grund der Umstellung (Schritt 5). Der Notarzt muss die Spritzenpumpe mit den neuen Werten manuell am Gerät starten (Schritt 6), da der TNA aufgrund der unzureichenden Netzqualität keinen Zugriff auf die Fernsteuerung der Spritzenpumpe hat. Das System stellt diesen Zustand parallel auf der Benutzerschnittstelle im Rettungswagen durch Anzeige "Nur überwachte Eingriffe" dar.

Daraufhin stabilisiert sich der Patient. Die Weiterfahrt des Rettungswagens bis zum Eintritt in den Stadtbereich geschieht unter reduzierter Mobilfunkverbindung.

Während der Schritte 3 - 6 werden keine Stammdaten übertragen, da sie eine niedrige Priorität haben und nicht in die verfügbare Netzwerkkapazität passen. Ein verbleibender Teil der Stammdaten wird daher nicht hochgeladen, bis sich die Verbindungsqualität verbessert. Ebenso werden keine Vergangenheitsdaten gesendet, da diese viel weniger relevant sind als Live-Daten, die sich auf die aktuelle Situation des Patienten und beispielsweise seine Reaktion auf die Einnahme des Medikaments über den Perfusor beziehen. Die Vergangenheitsdaten haben also eine niedrige Priorität und werden ebenfalls erst hochgeladen, wenn sich die Verbindungsqualität verbessert. Allerdings sammeln sich diese Vergangenheitsdaten an und erhöhen den damit verbundenen Datendruck auf 0,9.

Nach Eintritt in den Stadtbereich (Schritt 7), erhöht sich die verfügbare Netzwerkkapazität. Alle relevanten Services (Video, Audio, Vitalparameter-, 4-Kanal-EKG und EIT) werden wieder auf ein höheres FL gestellt und übertragen. Zudem werden Datenströme gesendet, welche während der früheren Fahrtabschnitte erfasst aber nicht gesendet wurden. Diese werden als ein einziger Service behandelt, der gemäß Tabelle 1 als "Vergangenheitsdaten" oder Archive bezeichnet wird. Die Vergangenheitsdaten werden dann auch an den Telenotarzt gesendet und zur Information über die weitere Behandlung verwendet. Ein Arzt im Krankenhaus ist somit über den Status des Patienten, die Gesamtmenge der bereits eingenommenen Medikamente und deren Wirksamkeit informiert. Eine angepasste Weiterbehandlung im Krankenhaus kann ohne Verzögerung erfolgen.

Fig. 1 zeigt eine schematische Darstellung eines Verfahrens gemäß einer bevorzugten Ausführungsform der Erfindung. Ein System gemäß einer bevorzugten Ausführungsform der Erfindung ist vorzugsweise dafür konfiguriert, die Verfahrensschritte auszuführen. Der Prozessor **10** des erfindungsgemäßen Systems kann verschiedene Untereinheiten umfassen, insbesondere einen Druckberechner **12,** einen Workflow-Manager **16,** einen Priorisierer **20** und einen Optimierer **24.** Der Prozessor kann auch einen Reduzierer **28** umfassen, der hier auch als Service-Gateway bezeichnet wird.

Der Prozessor **10** ist mit einer Mehrzahl von aktiven Datenerfassungsgeräten und Medizingeräten in einer mobilen Behandlungseinheit verbunden, wobei die Verbindung auch drahtlos sein kann. Der Prozessor empfängt Live-Daten **2** von den aktiven Datenerfassungsgeräten und kann Befehle an die Medizingeräte geben. Die Live-Daten **2** werden vorzugsweise von dem Prozessor auf einen Speicher gespeichert. Sollten diese Live-Daten **2** innerhalb einer vorgegebenen Zeit nicht an das Zentrum für telemedizinische Unterstützung übertragen, werden sie von dem Prozessor als Vergangenheitsdaten **6** betrachtet. Die Vergangenheitsdaten **6** werden als ein eigenständiger Service behandelt. Sollte der Prozessor **10** Zugriff auf Bestandsdaten **4** haben, werden diese als weiterer Service behandelt. Aus den Live-Daten **2,** Vergangenheitsdaten **6** und/oder Bestandsdaten **4** sowie einer optionalen Überprüfung der verfügbaren Medizingeräte erstellt der Prozessor eine vollständige Liste **14** aller verfügbaren Services.

Vorzugsweise umfasst der Prozessor **10** auch Mittel zur Vorhersage einer bevorstehenden Verbindungsqualität **32.** Dabei kann es sich um eine Verbindungsqualität handeln, die voraussichtlich in einer Minute, zwei Minuten, fünf Minuten oder mehr verfügbar sein wird. Alternativ kann dies auch in Form einer Entfernung definiert werden und kann eine Verbindungsqualität sein, die voraussichtlich in 1 km, 1,5 km, 2 km oder mehr verfügbar ist. Der Druckberechner **12** ist vorzugsweise dafür konfiguriert, einen "Datendruck" zu berechnen. Der Datendruck stellt vorzugsweise ein Verhältnis zwischen zu sendenden nicht-Live-Daten (d.h. Bestandsdaten **4** und Vergangenheitsdaten **6)** und einer erwarteten Sendekapazität zu einem künftigen Zeitpunkt dar. Vorzugsweise dient der Datendruck als Abstraktion dafür, wie dringend es zu einem gegebenen Zeitpunkt ist, Bestands- **4** oder Vergangenheitsdaten **6** über Live-Daten **2** zu priorisieren. Der Druckberechner **12** fügt dafür den Services von Bestands- **4** und Vergangenheitsdaten **6** ein MetaDatum "DatenDruckWert" hinzu. Vorzugsweise hat der Datendruck einen Wert von 0 oder mehr, wobei ein Datendruck von 0 bedeutet, dass keine Daten mehr gesendet werden müssen. Ein Datendruck über 1 bedeutet vorzugsweise, dass nicht mehr alle Daten gesendet werden können. Je höher der Wert ist, desto mehr Datendruck liegt an. Der Prozessor kann diese Angaben verwenden, um den Services von Bestandsdaten **4** und Vergangenheitsdaten **6** einen Prioritätswert zuzuweisen. Dies ist besonders nützlich, um zu erwartende Steigerungen der Netzwerkqualität zu nutzen oder um sicherzustellen, dass Bestandsdaten **4** und Vergangenheitsdaten **6** an das Zentrum für telemedizinische Unterstützung gesendet werden, bevor die Netzwerkqualität abfällt. Bei Verwendung des Druckberechners **12** kann die vollständige Liste aller möglichen Services **14** die verschiedenen Arten von Live-Daten **2,** die Bestandsdaten **4** und die Vergangenheitsdaten **6** umfassen, wobei die beiden letzteren auch ein Metadatum "Datendruck" umfassen.

Der Workflow-Manager **16** verfügt über die vollständige Liste der Services **14** sowie die Benutzereingaben **38** und jegliche Daten, welche durch die Kommunikationseinheit empfangen werden. Der Workflow-Manager **16** hat vorzugsweise ein semantisches Verständnis des medizinischen Geschehens. Beispielsweise umfasst der Workflow-Manager **16** eine Matrix, Korrelation oder ein Algorithmus, welche die verfügbaren Inputs (z.B. Live-Daten **2,** jegliche Bestandsdaten **4,** Vergangenheitsdaten **6** oder Benutzereingaben **38)** bewerten und einer medizinischen Situation (z.B. Polytrauma) zuordnen kann. Vorzugsweise ist der Workflow-Manager **16** auch so konfiguriert, dass er einen bestimmten medizinischen Verfahrensschritt (z.B. die Untersuchung auf innere Verletzungen) auf der Grundlage der Inputs identifiziert. Dies kann auf der Basis eines oder mehrerer Prozessmodelle erfolgen, auf die der Workflow-Manager **16** des Prozessors **10** Zugriff hat. Dabei kann es sich um eine weitere Matrix, Korrelation oder einen Algorithmus handeln, der so konfiguriert ist, dass er einen aktuellen oder nachfolgenden Schritt vorhersagt, der für die Versorgung eines Patienten erforderlich ist. Der Workflow-Manager **16** ist weiterhin dafür konfiguriert, auf Basis des medizinischen Verfahrensschritts, eine Menge von relevanten Services festzulegen und für jeden relevanten Service ein oder mehrere medizinisch sinnvolle FL zu identifizieren. Dies kann ebenfalls aufgrund einer Matrix, Korrelation oder Algorithmus' erfolgen. Vorzugsweise werden die vom Workflow-Manager **16** verwendeten Matrizen, Korrelationen oder Algorithmen auf der Grundlage von medizinischem Expertenwissen vorbereitet, das durch maschinelles Lernen kontinuierlich verbessert werden kann. Das Ergebnis ist eine Liste von medizinisch sinnvollen FL **18,** die für den identifizierten medizinischen Verfahrensschritt relevant sind. Bei allen medizinisch sinnvollen FL **18** handelt es sich um Ausführungsformen der vollständigen Liste der verfügbaren Services **14,** wobei nicht jeder verfügbare Service repräsentiert werden muss.

Die Liste der medizinisch relevanten und medizinisch sinnvollen FL **18** wird dem Priorisierer **20** zur Verfügung gestellt. Der Priorisierer **20** erstellt aus der sinnvollen Menge der FL **18** eine priorisierte Liste, beispielsweise durch Hinzufügen eines Prioritätswert-Metadatum zu jedem FL. Bei der Priorisierung kann es sich ebenfalls um eine Sortierung, Klassifizierung oder multi-dimensionale Bewertung handeln. Durch die Verteilung dieser Prioritäten wird vorzugsweise die Wichtung der Services festgelegt, die für die Auswahl eines bestimmten FL pro Service im Optimierer **24** entscheidend ist. Beispielsweise ist der Service EKG zurzeit sehr wichtig. Deswegen werden alle verfügbaren FL des Services EKG höher priorisiert als das höchste FL anderer Services. Sollte ein Benutzer mit der vom Priorisierer **20** vorgenommenen Priorisierung nicht einverstanden sein, kann diese auch durch Benutzereingaben **38** geändert werden, sofern der Benutzer dazu berechtigt ist.

Vorzugsweise verfügt der Priorisierer **20** über eine Matrix, eine Korrelation oder ein Algorithmus, wobei jedem FL ein Prioritätswert anhand des Verfahrensschritts innerhalb der medizinischen Situation und dem Behandlungsstatus des Patienten zugewiesen wird. In einer bevorzugten Variante wird eine statische Matrix im Voraus erzeugt, die für jedes FL der Services, jeden medizinischen Verfahrensschritt und für Gruppen von Eingangsdaten einen Eintrag enthält, welches FL zu wählen ist. Diese Matrix würde den kompletten Suchraum abdecken und würde dafür eine vollständige Validierung zulassen. Erstellt würde sie vorzugsweise durch Zusammenlegen von Know-How von Medizinern unterschiedlicher Fachbereiche sowie Experten des Rettungswesens und der Informatik. In einer weiteren bevorzugten Variante wird stattdessen eine weniger umfassende Basismatrix für eindeutige oder grundlegende Fälle vorab erstellt. Der Priorisierer **20** umfasst in dieser Variante vorzugsweise ein Entscheidungsmechanismus, wodurch weitere Fälle, die noch nicht in der Basismatrix umfasst sind, "on-the-fly" erzeugt werden. Der Basismatrix kann dann kontinuierlich durch maschinelles Lernen ergänzt und aktualisiert werden.

Ein Optimierer **24** im Prozessor **10** hat auf die priorisierte Liste **22** der medizinisch sinnvollen FL sowie auf eine aktuelle Verbindungsqualität, insbesondere auf den aktuellen Wert mindestens eines Qualitätsparameters, Zugriff. Die aktuelle Verbindungsqualität kann eine erwartete Verbindungsqualität **32** sein. Vorzugsweise ist diese aber eine aktuell gemessene Verbindungsqualität **8,** vorzugsweise von einem Netzwerkqualitätsüberwacher. Letzteres ist zuverlässiger, da die tatsächliche Netzwerkqualität nicht nur vom verfügbaren Dienst abhängt, sondern u.a. auch vom Verkehrsaufkommen und damit von der Anzahl der anderen Nutzer. Der Optimierer **24** ist vorzugsweise dafür konfiguriert, ein mehrdimensionales bzw. geschachteltes Rucksackproblem zu lösen und damit eine optimierte Liste der FL **26** zu erstellen, welche in die verfügbare Netzwerkkapazität passen. Bei der optimierten Liste der FL **26** wird jeder Service höchstens einmal durch ein bestimmtes FL vertreten.

Als vereinfachtes Beispiel kann davon ausgegangen werden, dass der Workflow-Manager **16** für jeden Service nur ein FL als medizinisch sinnvoll erachtet und der Priorisierer **20** eine priorisierte Liste dieser FL bereitstellt. Die Aufgabe des Optimierers bestünde darin, die FL beginnend mit den wichtigsten auszuwählen, bis ein Punkt erreicht ist, an dem bei der verfügbaren Netzwerkqualität keine Daten mehr übertragen werden können. Mit anderen Worten: der Optimierer würde im Fall einer schwachen Netzwerkqualität ggf. Services vollständig von der Datenübertragung ausschließen.

Schließlich führt ein Reduzierer **28,** über den alle an das Zentrum für telemedizinische Unterstützung zu übertragenden Daten **40** geleitet werden, ggf. die Reduzierung der Services durch, um reduzierte Datenströme **30** bereitzustellen. Die Kommunikationseinheit übermittelt die reduzierten Services **30** an das Zentrum für telemedizinische Unterstützung.

### Bezugszeichenliste

- **2**: Live-Daten
- **4**: Bestandsdaten
- **6**: Vergangenheitsdaten
- **8**: Gemessene Verbindungsqualität
- **10**: Prozessor
- **12**: Druckberechner
- **14**: Vollständige Menge der Services
- **16**: Workflow Manager
- **18**: Medizinisch sinnvolle Menge der FL der Services
- **20**: Priorisierer
- **22**: Priorisierte Liste der FL
- **24**: Optimierer
- **26**: Optimierte Liste der FL
- **28**: Reduzierer
- **30**: Ausgewählte und ggf. reduzierte Services
- **32**: Erwartete Verbindungsqualität
- **38**: Benutzereingaben
- **40**: Medinisch relevante Daten

## Patentansprüche

1. System zur Kommunikation von medizinisch relevanten Daten zwischen einer mobilen Behandlungseinheit und einem Zentrum für telemedizinische Unterstützung, **dadurch gekennzeichnet, dass**
das System ein oder mehrere Datenerfassungsgeräte, eine oder mehrere Benutzerschnittstellen, einen Prozessor (10) und eine Kommunikationseinheit in der mobilen Behandlungseinheit umfasst,
wobei die Datenerfassungsgeräte für die Erfassung von medizinisch relevanten Daten (40) konfiguriert sind,
die eine oder mehreren Benutzerschnittstellen für die Eingabe und/oder Wiedergabe von medizinisch relevanten Daten (40) konfiguriert sind,
der Prozessor (10) dafür konfiguriert ist:
- anhand von Eingangsdaten (2, 4, 6, 38) von dem einen oder mehreren Datenerfassungsgeräten, von der einen oder mehreren Benutzerschnittstellen und/oder von der Kommunikationseinheit eine medizinische Situation zu identifizieren,
- auf Basis der medizinischen Situation und der Eingangsdaten (2, 4, 6, 38) einen medizinischen Verfahrensschritt und die dafür benötigten Services zu identifizieren,
- ein oder mehrere medizinisch sinnvolle diskrete Feature Levels (FL) (18) für jeden benötigten Service innerhalb des medizinischen Verfahrensschritts abzurufen,
- eine Priorisierung der FL (22) entsprechend des medizinischen Verfahrensschrittes durchzuführen,
- mindestens einen Qualitätsparameter (8) einer verfügbaren Netzwerkverbindung zwischen der Kommunikationseinheit und dem Zentrum für telemedizinische Unterstützung zu identifizieren,
- zu übermittelnde Services mit einem bestimmten FL (26) unter Berücksichtigung der Priorisierung und des Qualitätsparameters der verfügbaren Netzwerkverbindung auszuwählen,
- die ausgewählten Services optional auf ein bestimmtes FL zu reduzieren,
wobei die Kommunikationseinheit dafür konfiguriert ist, die ausgewählten Services zum Zentrum für telemedizinische Unterstützung zu übermitteln und/oder Daten von diesem zu empfangen.

2. System zur Kommunikation von medizinisch relevanten Daten gemäß Anspruch 1 **dadurch gekennzeichnet, dass**
die Priorisierung für jeweils eine Vielzahl von FL für jeden benötigten Service mithilfe einer dreidimensionalen Matrix durchgeführt wird, wobei jedem FL eines Services anhand einer Korrelation
- des FL,
- des Verfahrensschritts der medizinischen Situation und
- der Eingangsdaten
ein Prioritätswert zugeordnet wird.

3. System zur Kommunikation von medizinisch relevanten Daten gemäß dem vorherigen Anspruch
**dadurch gekennzeichnet, dass**
die Priorisierung durch Benutzereingaben (38) über die eine oder mehreren Benutzerschnittstellen beeinflussbar ist, wobei vorzugsweise die Benutzereingaben (38) die definierte Priorisierung durch eine abweichende Priorisierung ersetzt.

4. System zur Kommunikation von medizinisch relevanten Daten gemäß einem der vorherigen Ansprüche
**dadurch gekennzeichnet, dass**
die Auswahl der FL der zu übermittelnden Services durch Erstellung einer optimierten Liste von FL (26) erfolgt, wobei die optimierte Liste vorzugsweise durch Lösung eines mehrdimensionalen oder geschachtelten Rucksackproblems erstellt wird, wobei für jeden zu übermittelnden Service jeweils ein FL für die Übermittlung ausgewählt wird.

5. System zur Kommunikation von medizinisch relevanten Daten gemäß einem der vorherigen Ansprüche
**dadurch gekennzeichnet, dass**
der Prozessor (10) für eine Reduktion auf ein ausgewähltes FL für jeden Service konfiguriert ist, wobei die Reduktion vorzugsweise eine Reduktion einer Bandbreite, einer Bildrate, einer Auflösung, einer Abtastrate, einer Frequenz, ein Beschneiden und/oder ein Komprimieren der Daten umfasst.

6. System zur Kommunikation von medizinisch relevanten Daten gemäß einem der vorherigen Ansprüche
**dadurch gekennzeichnet, dass**
die zu übermittelnden Services (26) aus der folgenden Gruppe ausgewählt sind: ein Audiogespräch, ein schriftliches Gespräch, ein Foto, eine Fotobeschreibung, ein Video, ein Ultraschallstream, ein CT-Stream, ein holografischer Stream, ein AR-Stream, Pulsmessungen, EKG-Messungen, EIT-Messungen, Blutzuckermessungen, Blutdruckmessungen, Atemalkoholspiegel, Temperaturmessungen, Sauerstoffsättigungsmessungen, CO2-Ausatemgehaltsmessungen, Herzfrequenzmessungen, Atemfrequenzmessungen, Bestandsdaten und/ oder Vergangenheitsdaten.

7. System zur Kommunikation von medizinisch relevanten Daten gemäß einem der vorherigen Ansprüche
**dadurch gekennzeichnet, dass**
der Prozessor (10) ferner so konfiguriert ist, dass er Bestandsdaten (4) von der einen oder mehreren Benutzerschnittstellen, der Kommunikationseinheit oder dem einen oder mehreren Datenerfassungsgeräten empfängt, und wobei die Bestandsdaten vorzugsweise Stammdaten eines Patienten, eine Fallbeschreibung, eine Anamnese, Risikofaktoren, eine voraussichtliche Ankunftszeit, eine Einsatzhistorie, ein cABCDE-Schema und/oder Berichtsdaten eines Unfalls umfassen.

8. System zur Kommunikation von medizinisch relevanten Daten gemäß einem der vorherigen Ansprüche
**dadurch gekennzeichnet, dass**
das System einen Netzwerkqualitätsüberwacher zur Überwachung des Qualitätsparameters (8) der verfügbaren Netzwerkverbindung in der mobilen Behandlungseinheit umfasst.

9. System zur Kommunikation von medizinisch relevanten Daten gemäß einem der vorherigen Ansprüche
**dadurch gekennzeichnet, dass**
der Qualitätsparameter (8) der verfügbaren Netzwerkverbindung ausgewählt ist aus Bandbreite, Latenz und/oder Jitter.

10. System zur Kommunikation von medizinisch relevanten Daten gemäß einem der vorherigen Ansprüche
**dadurch gekennzeichnet, dass**
der Prozessor (10) über eine Netzwerkqualitätskarte verfügt und dafür konfiguriert ist, mindestens einen Qualitätsparameter (32) der verfügbaren Netzwerkverbindung anhand der Netzwerkqualitätskarte vorherzusagen.

11. System zur Kommunikation von medizinisch relevanten Daten gemäß dem vorherigen Anspruch
**dadurch gekennzeichnet, dass**
der Prozessor (10) weiterhin dafür konfiguriert ist, eine Fahrtroute der mobilen Behandlungseinheit anhand einer gespeicherten Netzwerkqualitätskarte und/oder der medizinischen Situation zu berechnen, wobei die berechnete Route vorzugsweise über die eine oder mehreren Benutzerschnittstellen angezeigt wird.

12. Verfahren zur Kommunikation von medizinisch relevanten Daten zwischen einer mobilen Behandlungseinheit und einem Zentrum für telemedizinische Unterstützung,
**dadurch gekennzeichnet, dass**
das Verfahren die folgenden Schritte umfasst:
- Identifizieren einer medizinischen Situation anhand von Eingangsdaten (2, 4, 6, 38) von einem oder mehreren Datenerfassungsgeräten, von einer oder mehreren Benutzerschnittstellen und/oder von einer Kommunikationseinheit in der mobilen Behandlungseinheit,
- Identifizieren eines medizinischen Verfahrensschrittes und der dafür benötigten Services auf Basis der medizinischen Situation und der Eingangsdaten,
- Abrufen von diskreten medizinisch sinnvollen Feature Levels (FL) (18), für jeden benötigten Service innerhalb des medizinischen Verfahrensschrittes,
- Priorisierung der FL (22) entsprechend des medizinischen Verfahrensschrittes,
- Identifizieren mindestens eines Qualitätsparameters (8) einer verfügbaren Netzwerkverbindung zwischen der Kommunikationseinheit und dem Zentrum für telemedizinische Unterstützung,
- Auswahl der zu übermittelnden Services mit einem bestimmten FL (26) unter Berücksichtigung der Priorisierung und des Qualitätsparameters der verfügbaren Netzwerkverbindung,
- Optionale Reduktion der ausgewählten Services auf ein bestimmtes FL,
- Übermitteln der ausgewählten Services zum Zentrum für telemedizinische Unterstützung und/oder Empfangen von Daten vom Zentrum für telemedizinische Unterstützung,
wobei das Verfahren vorzugsweise durch ein System gemäß einem der Ansprüche 1 - 11 durchgeführt wird.
